# EUROPEAN PATENT SPECIFICATION

(11) **EP 3 028 044 B1**
(45) Date of publication and mention of the grant of the patent: **19.06.2019**
(21) Application number: 14832661.4
(22) Date of filing: 29.07.2014
(51) Int. Cl.: G01N 33/53, G01N 33/68

(54) **NON-GLYCOSYLATED SUPAR BIOMARKERS AND USES THEREOF**
NICHT-GLYCOSYLIERTE SUPAR-BIOMARKER UND VERWENDUNGEN DAVON
BIOMARQUEURS SUPAR NON GLYCOSYLÉS ET UTILISATIONS DE CEUX-CI

(30) Priority: 01.08.2013 US 201361861226 P
(43) Date of publication of application: 08.06.2016
(73) Proprietor: The General Hospital Corporation, Boston, MA 02114 (US); Rush University Medical Center, Chicago, IL 60612 (US)
(72) Inventor: SEVER, Sanja, Brookline, Massachusetts 02446 (US); REISER, Jochen, Hinsdale, Illinois 60521 (US)
(74) Representative: Kunz, Herbert
(86) International application number: PCT/US2014/048568
(87) International publication number: WO 2015/017386

(56) References cited:
- WO-A1-2010/054189
- WO-A2-02/058714
- WO-A2-2006/094828
- WO-A2-2012/154218
- WO-A2-2013/066879
- BEHRENDT N ET AL: "THE HUMAN RECEPTOR FOR UROKINASE PLASMINOGEN ACTIVATOR. ÖNH2-TERMINAL AMINO ACID SEQUENCE AND GLYDOSYLATION VARIANTS", JOURNAL OF BIOLOGICAL CHEMISTRY, AMERICAN SOCIETY FOR BIOCHEMISTRY AND MOLECULAR BIOLOGY, US, vol. 265, no. 11, 15 April 1990 (1990-04-15), pages 6453-6460, XP000604990, ISSN: 0021-9258
- GARDSVOLL H ET AL: "Characterization of low-glycosylated forms of soluble human urokinase receptor expressed in Drosophila Schneider 2 cells after deletion of glycosylation-sites", PROTEIN EXPRESSION AND PURIFICATION, ACADEMIC PRESS, SAN DIEGO, CA, vol. 34, no. 2, 1 April 2004 (2004-04-01), pages 284-295, XP004493236, ISSN: 1046-5928, DOI: 10.1016/J.PEP.2003.12.002
- CHANGLI WEI ET AL: "Circulating urokinase receptor as a cause of focal segmental glomerulosclerosis", NATURE MEDICINE, vol. 17, no. 8, 31 July 2011 (2011-07-31), pages 952-960, XP055171823, ISSN: 1078-8956, DOI: 10.1038/nm.2411
- DETLEF SCHLÖNDORFF: "Are serum suPAR determinations by current ELISA methodology reliable diagnostic biomarkers for FSGS?", KIDNEY INTERNATIONAL, vol. 85, no. 3, 1 March 2014 (2014-03-01), pages 499-501, XP055319242, LONDON, GB ISSN: 0085-2538, DOI: 10.1038/ki.2013.549
- J. REISER ET AL: "Soluble Urokinase-Type Plasminogen Activator Receptor in FSGS: Stirred but Not Shaken", JOURNAL OF THE AMERICAN SOCIETY OF NEPHROLOGY., vol. 25, no. 8, 1 May 2014 (2014-05-01), pages 1611-1613, XP055318975, US ISSN: 1046-6673, DOI: 10.1681/ASN.2014030257
- THUNO, M ET AL.: 'suPAR: The Molecular Crystal Ball.' DIS MARKERS. vol. 27, no. 3, 2009, pages 157 - 72, XP055314721
- IANNI, M ET AL.: 'Altered Glycbsylation Profile Of Purified Plasma ACT From Alzheimer's Disease.' IMMUNITY & AGEING. vol. 7, no. SUPPL., 2010, page S6, XP021089672

## Description

### BACKGROUND OF THE INVENTION

Proteinuria soluble urokinase plasminogen-type activator receptor (suPAR) has been suggested as a causative factor of proteinuria in kidney disorders such as diabetic nephropathy and focal segmental glomerulosclerosis (FSGS). For example, it has been shown previously that about two-thirds of patients with primary FSGS and recurrent FSGS had increased serum levels of suPAR. Using *in vitro* and *in vivo* studies, it has been shown that suPAR activates avβ3 integrin on podocyte foot processes (FPs), which in turn induces proteinuria in mice. However, suPAR levels are elevated in a number of diseases including cancer and infection that are not associated with proteinuria. Thus, the relevance of suPAR analyte levels to proteinuria is still unclear. The present application addresses these issues.

WO 2010/054189 A1 describes compositions which specifically block urokinase receptor (uPAR) in podocytes, compositions which modulate or block soluble urokinase receptor (suPAR), and compositions which modulate pathways in which uPAR is associated with, to protect against renal diseases or disorders.

WO 2012/154218 A2 describes that FSGS pathogenesis can be stopped or slowed by ex vivo removal of suPAR from a subject's circulation and that removal may be measured by comparing the level (e.g., amount or concentration) of suPAR before and after such treatment.

### SUMMARY OF THE INVENTION

Proteinuria markers and methods for their use are provided. These markers find many uses, including in diagnosing proteinuria, prognosing proteinuria, and treating proteinuria. In addition, reagents, devices and kits thereof that find use in practicing the subject methods are provided.

The present invention is defined by the independent claims. The dependent claims depict other embodiments of the invention.

### BRIEF DESCRIPTION OF THE DRAWINGS

The invention is best understood from the following detailed description when read in conjunction with the accompanying drawings. The patent or application file contains at least one drawing executed in color. Copies of this patent or patent application publication with color drawing(s) will be provided by the Office upon request and payment of the necessary fee. It is emphasized that, according to common practice, the various features of the drawings are not to-scale. On the contrary, the dimensions of the various features are arbitrarily expanded or reduced for clarity. Included in the drawings are the following figures.

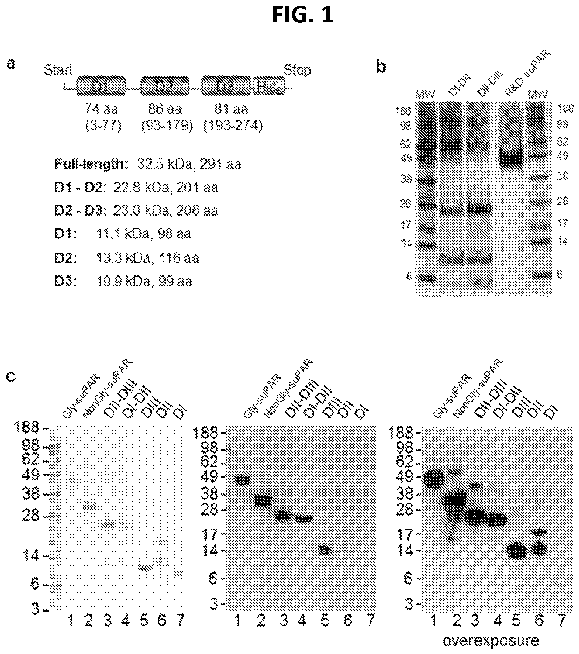

**Figure 1A** depicts the C-terminal His-tagged NonGly-suPAR protein fragments used in the domain studies.
**Figure 1B** shows a silver stain of an SDS-Page gel on which the protein fragments depicted in 2A and suPAR protein (R & D) were separated. Note the high degree of purity of the protein in the samples.
**Figure 1C** shows expression of NonGly-suPAR fragments in E. coli. Panel 1 (leftmost panel): Coomassie staining of total bacterial lysates. Panel 2 (center panel): Western blot analysis using anti-PLAUR ab from Sigma. Panel 3 (right-most panel): overexposure of panel 2. Lane 1, Gly-suPAR DI-DIII from R&D; lane 2, NonGly-suPAR DI-DIII; lane 3, DII-DIII; lane 4, DI-DII; lane 5, DIII; lane 6, DII; lane 7, DI.

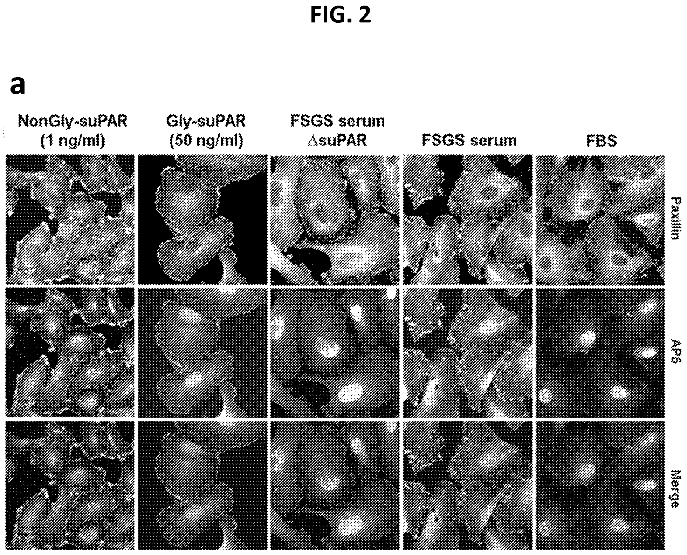

**Figure 2A** shows that plasma from FSGS patients ("FSGS serum") activates αᵥβ₃ integrin, and depletion of suPAR from FSGS plasma using antibody ATN615 ("FSGS serum ΔsuPAR") ameliorates αᵥβ₃ integrin activation. The figure also shows that wild type fully glycosylated suPAR (Gly-suPAR) is not a potent activator of αᵥβ_{3,} in contrast to non-glycosylated suPAR (NonGly-suPAR). Notice that more potent activation is observed by addition of 1 ng/ml of NonGly-suPAR, then in the presence of 50 ng/ml of Gly-suPAR. Thus, NonGly suPAR is potent activator of integrin at physiological levels.

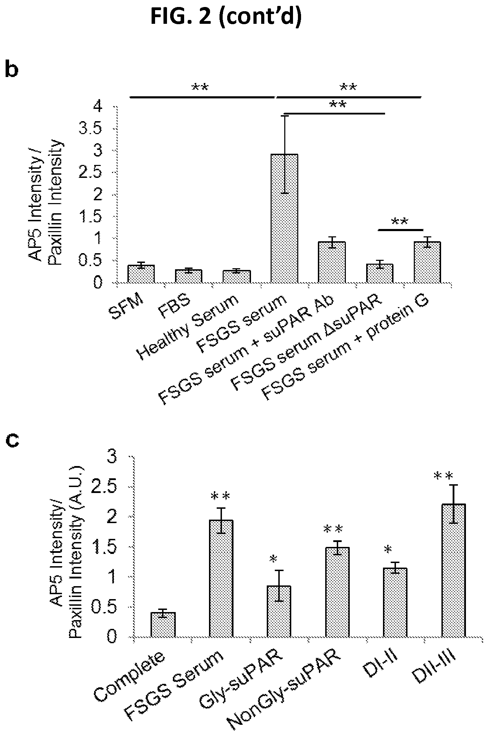

**Figure 2B** provides the quantitation of results in Figure 2A (panels 1-3). At least 30 cells were counted per condition. Data represent mean ±S.E.M. n=3.
**Figure 2C** provides the quantitation of results in 2A (panels 4, 5) in addition to quantification of αᵥβ₃ integrin by the non-glycosylated fragments of suPAR (DI-DII and DII-DIII). At least 30 cells were counted per condition. Data represent mean ± S.E.M, n=3.

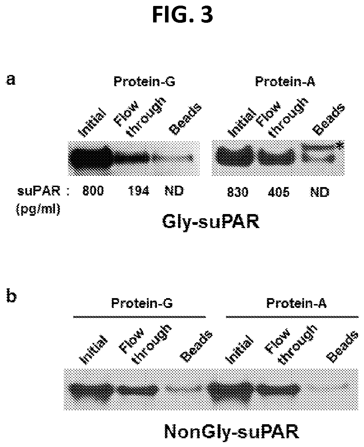

**Figures 3A and 3B** show that both Gly-suPAR (panel A) and NonGly-suPAR bind to the protein-G and protein-A. In panel 3A, *Protein A (65 kDa) that was released during elution step.

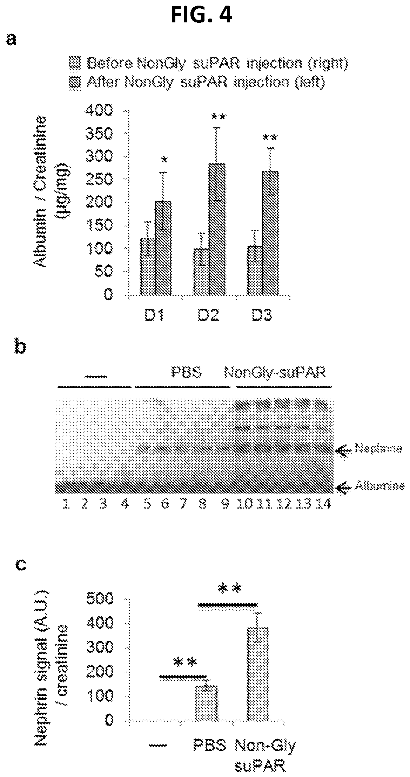

**Figures 4A-4C** show that nonGly-suPAR induces proteinuria in wild type mice. Figure 4a, bar graphs showing levels of albuminuria before and after injection of NonGly-suPAR in mice. Data represent mean ±S.D, n=6 animals. Figure 4b shows appearance of nephrin in urine of animals after they were injected with NonGly-suPAR (lanes 10-14). Injection with PBS was used as a control for stress upon injection, which also injures podocytes (notice appearance of nephrin signal in lanes 5-9). A loss of nephrin into urine (nephrinuria) has been proposed as early diagnostic tool for podocyte injury since it proceeds microalbuminuria in humans (Ziyadeh FN and Wolf G, 2008, Current diabetes reviews 4 (1), 39-45). Figure 4C provides the quantitation of results in 4B.

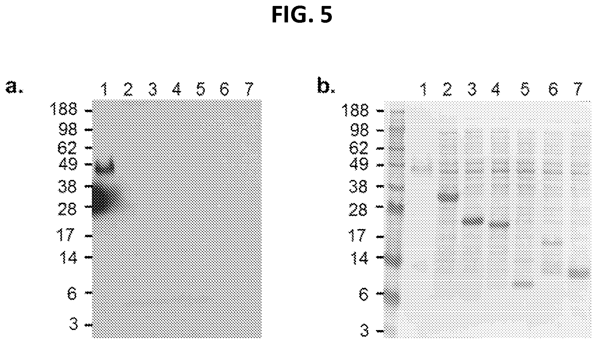

**Figures 5A and 5B** demonstrate that the anti-suPAR antibody (R&D) used in ELISA experiments recognizes only Gly-suPAR. Panel a: Western blot. Panel b: Coomassie Blue. Lane 1. R&D His-tagged full-length suPAR expressed in mammalian cells, 500 ng/lane; lane 2. His-tagged full-length suPAR expressed in *E. coli;* lane 3. His-tagged D2-D3 suPAR expressed in *E. coli;* lane 4. His-tagged D1-D2 suPAR expressed in *E. coli;* lane 5. His-tagged D1 suPAR expressed in *E. coli;* lane 6. His-tagged D2 suPAR expressed in *E. coli;* lane 7. His-tagged D3 suPAR expressed in *E. coli.* Signals developed using SuperSignal®West Fempto Maximum Sensitivity Substrate from Thermo Scientific.

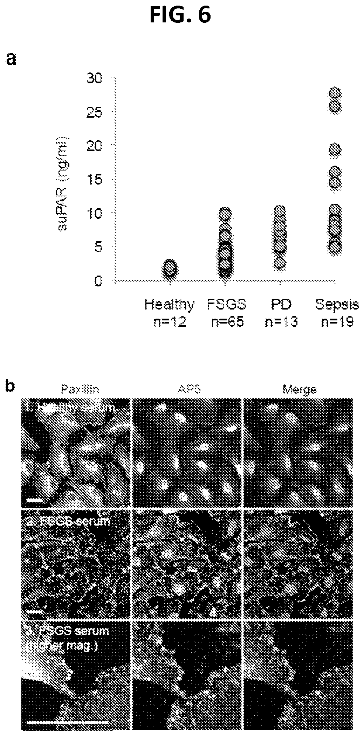

**Figures 6A-6E** provide data showing that suPAR levels in plasma do not correlate with β₃ integrin activation. Panel A: sera of patients with FSGS exhibited increased suPAR levels, whereas suPAR levels were even more pronounced in patients on peritoneal dialysis, or those with sepsis. Panel B: measurement of the ratios of levels of activated integrin (AP5 staining) to levels of total amount of focal adhesions (FAs, determined by Paxillin staining). Panel C: A number of sera induced potent β₃ integrin activation, but no statistically significant correlation was found between suPAR levels and levels of integrin activation. Panels D and E: β₃ integrin activation was not induced by the sera of patients on peritoneal dialysis (Panel D), or those with sepsis (Panel E).

### DETAILED DESCRIPTION OF THE INVENTION

Methods and compositions are provided for providing a proteinuria assessment. Aspects of the methods comprise evaluating the level of non-glycosylated suPAR in a sample from a subject. In addition, reagents, devices and kits thereof that find use in practicing the subject methods are provided. These and other objects, advantages, and features of the invention will become apparent to those persons skilled in the art upon reading the details of the compositions and methods as more fully described below.

Before the present methods and compositions are described, it is to be understood that this invention is not limited to particular method or composition described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and preferred methods and materials are now described. It is understood that the present disclosure supercedes any disclosure of publication to the extent there is a contradiction.

As will be apparent to those of skill in the art upon reading this disclosure, each of the individual embodiments described and illustrated herein has discrete components and features which may be readily separated from or combined with the features of any of the other several embodiments without departing from the scope of the present invention. Any recited method can be carried out in the order of events recited or in any other order which is logically possible.

It must be noted that as used herein and in the appended claims, the singular forms "a", "an", and "the" include plural referents unless the context clearly dictates otherwise. Thus, for example, reference to "a cell" includes a plurality of such cells and reference to "the peptide" includes reference to one or more peptides and equivalents thereof, e.g. polypeptides, known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### COMPOSITIONS

In some aspects of the invention, proteinuria biomarkers are provided. By "proteinuria", it is meant the presence of excessive amounts of serum protein in the urine. By a "proteinuria biomarker" or "proteinuria marker", it is meant a molecular entity whose representation in a sample (e.g., a blood sample or a urine sample) is associated with, i.e., correlates with, proteinuria. For example, a proteinuria marker may be differentially represented, i.e. represented at a different level, or abundance, in a sample from an individual that will develop or has developed proteinuria as compared to a healthy individual. Proteinuria markers find many uses, for example in diagnosing proteinuria in a subject, prognosing a subject's proteinuria, monitoring proteinuria in a subject, determining a proteinuria treatment for a subject, and in research, e.g. in the discovery of new agents for the treatment of proteinuria. These and other applications are described in greater detail below.

In some aspects of the invention, the subject proteinuria biomarkers are suPAR analytes. By "suPAR", it is meant the polypeptide that is the soluble form of the membrane-bound receptor for urokinase (uPA), urokinase-type plasminogen activator receptor (uPAR). By a "suPAR analyte" it is meant a suPAR polypeptide or variant or fragment thereof. The terms "protein" and "polypeptide" as used in this application are interchangeable. "Polypeptide" refers to a polymer of amino acids (amino acid sequence) and does not refer to a specific length of the molecule. Thus peptides and oligopeptides are included within the definition of polypeptide. This term also refers to or includes post-translationally modified polypeptides, for example, glycosylated polypeptide, acetylated polypeptide, phosphorylated polypeptide and the like. Included within the definition are, for example, polypeptides containing one or more analogs of an amino acid, polypeptides with substituted linkages, as well as other modifications known in the art, both naturally occurring and non-naturally occurring.

uPAR, also known as "CD87", is a glycosylphosphatidylinositol (GPI)-anchored cell-surface protein encoded by the PLAUR gene. Relevant sequences include Genbank Accession Nos. NM_002659.3 (nucleic acid sequence encoding uPAR polypeptide, SEQ ID NO:1), and NP_002650.1 (uPAR mature polypeptide, SEQ ID NO:2). uPAR comprises three domains denoted uPAR(I) (consisting essentially of about amino acids 2-77 of SEQ ID NO:2); uPAR(II) (consisting essentially of about amino acids 93-179 of SEQ ID NO:2), and uPAR(III) (consisting essentially of about amino acids 193 - 274 of SEQ ID NO:2), uPAR(III) being anchored to the cell membrane by a juxtamembrane GPI domain (Ploug et al, 1991; crystal structure disclosed in Llinas et al, 2005). By "consisting essentially of", it is meant a limitation of the scope of composition or method described to the specified materials or steps that do not materially affect the basic and novel characteristic(s) of the subject invention. For example, a domain "consisting essentially of" a disclosed sequence has the amino acid sequence of the disclosed sequence plus or minus about 10 amino acid residues at the boundaries of the sequence based upon the sequence recited, e.g. about 10 resides, 9 residues, 8 residues, 7 residues, 6 residues, 5 residues, 4 residues, 3 residues, 2 residues or about 1 residue less than the recited bounding amino acid residue, or about 1 residue, 2 residues, 3 residues, 4 residues, 5 residues, 6 residues, 7 residues, 8 residues, 9 residues, or 10 residues more than the recited bounding amino acid residue.

It has been observed that mature full-length uPAR can be cleaved by uPA in a first linker region (i.e. between domains I and II), liberating uPAR(I) (Hoyer-Hansen et al, 1992; Zhou et al, 2000). Additionally or alternatively, uPAR can be cleaved by proteases at its GPI anchor, shedding suPAR(I-III) (molecular weight of approximately 30.7 kDa) or -- if already cleaved at the first linker region by uPA -- shedding suPAR(II-III) (molecular weight of approximately 21 kDa) from the cell surface (Piironen et al. Specific immunoassays for detection of intact and cleaved forms of the urokinase receptor. Clin. Chem. 2004;50:2059-68; Hoyer-Hansen and Lund. Urokinase receptor variants in tissue and body fluids. Adv. Clin. Chem 2007; 44:65-102). Thus, exemplary proteinuria markers include suPAR(I-III), suPAR(II-III), uPAR(I), and fragments thereof, i.e., polypeptide and peptide fragments.

Of particular interest in the subject compositions are suPAR analytes that are nonglycosylated. By a nonglycosylated ("nonGly-suPAR") suPAR analyte, it is meant a suPAR polypeptide or variant or fragment thereof that is hypoglycosylated. By glycosylation, it meant the process by which a molecule, e.g. protein, lipid, or other organic molecule, is co-translationally or post-translationally modified to comprise carbohydrate, or "glycan", moieties. Glycosylation is an enzyme-directed process that occurs in a number of different locations in the cell. For example, the majority of proteins synthesized in the rough ER undergo glycosylation. Glycosylation may also occur in the cytoplasm and nucleus. A number of different mechanisms for glycosylation exist, all of which encompassed by the present disclosure, which will result in the sugar molecule being attached to different functional groups. These include N-linked glycosylation, in which glycan is bound to the amino group of asparagine, and which typically takes place in the ER; O-linked glycosylation, in which monosaccharides are bound to the hydroxyl group of serine or threonine, and which typically takes place in the ER, Golgi, cystosol and nucleus; glypiation, in which glycan core links a phospholipid and a protein; C-linked glycosylation, in which mannose is bound to the indole ring of tryptophan; and phosphoglycosylation, in which a glycan is bound to a serine via phosphodiester bond. Proteins are often glycosylated at multiple sites with different glycosidic linkages, depending on enzyme availability, amino acid sequence, and protein conformation. By hypogylcosylated, it is meant being incompletely glycosylated, i.e. having less glycan moieties attached to the molecule (e.g., suPAR(I-III), suPAR(II-III), and/or suPAR(I), or any isoforms thereof), than if fully glycosylated.

The glycosylation pattern of uPAR has been determined by matrix assisted laser desorption ionization and electrospray ionization mass spectrometry (Ploug, et al. (1998). Glycosylation profile of a recombinant urokinase-type plasminogen activator receptor expressed in Chinese hamster ovary cells. The Journal of Biol. Chem. 273, 13933-13943). Without being bound by theory, it is believed that of the five potential attachment sites for N-linked carbohydrate in uPAR, only four are utilized, the tryptic peptide derived from domain III containing Asn233 being quantitatively recovered without carbohydrate. The remaining four attachment sites were shown to exhibit site-specific microheterogeneity of the asparagine-linked carbohydrate. The glycosylation on Asn52 (domain I) and Asn172 (domain II) is dominated by the smaller bi-antennary complex-type oligosaccharides, while Asn162 (domain II) and Asn200 (domain III) predominantly carry tri- and tetra-antennary complex-type oligosaccharides. Thus, fully glycosylated full-length suPAR typically contains N-linked carbohydrates at 4 sites: one in domain I, two in domain II, and one in domain III.

According to certain aspects, nonGly-suPAR(I-III) is glycosylated at less than four putative glycosylation sites, e.g., 3, 2, 1 or no sites. In certain aspects, nonGly-suPAR(II-III) is glycosylated at less than three putative glycosylation sites, e.g., 2, 1 or no sites. According to certain embodiments, nonGly-suPAR(I) is not glycosylated at any putative glycosylation sites. Any of the above hypoglycosylated suPAR molecules, alone or in any desired combination, may be utilized (e.g., as biomarkers) in the methods, devices and kits of the present disclosure.

The inventors of the present disclosure have discovered that elevated levels of nonglycosylated suPAR in blood are associated with a proteinuria phenotype and can induce proteinuria. For example, an increase of about 1.5-fold or more, e.g. 2-fold or more, 2.5-fold or more, 3-fold or more, 4-fold or more, or 5-fold or more in nonGly-suPAR analyte levels over nonGly-suPAR analyte levels observed in healthy individuals is indicative of proteinuria or an increased risk in developing proteinuria. As such, nonGly-suPAR analyte, i.e. nonglycosylated full length suPAR and nonglycosylated suPAR variants and fragments thereof, e.g. as described above, may be employed as proteinuria markers.

For example, the subject nonGly-suPAR analytes may have 25% less glycan moieties than fully glycosylated species or less, e.g. 30% less, 40% less, 50% less (i.e. half as many), 60%, 70%, 80%, or 90% less glycan/sugar moieties than the fully glycosylated suPAR. Put another way, nonGly-suPAR analytes of the subject disclosure may comprise75% as much glycan or less as fully glycosylated suPAR, e.g. 70% as much glycan or less, 60% as much glycan or less, 50% as much glycan (i.e. half as much) or less, 40% as much glycan or less, 30% as much glycan or less, 25% as much 20% as much glycan or less, or 10% as much glycan or less of fully glycosylated suPAR. Thus, for example, if fully glycosylated full-length glycan contains four N-linked carbohydrates, nonGly-suPAR variant may contain three N-linked glycans, sometimes two N-linked glycans or one N-linked glycan, or no N-linked glycans. More usually, the nonGly-suPAR analyte comprises no glycan moieties, i.e. it is substantially free of glycan/sugar moieties.

Any convenient method may be used to determine if or confirm that the suPAR analyte that is detected is a non-Gly suPAR analyte. For example, the extent, if any, to which a protein is glycosylated may be evaluated by chemically restructuring the glycan groups with periodic acid. Periodic acid oxidizes vicinal hydroxyls on sugars (especially sialic acid) to aldehydes or ketones, which are then reactive to multiple dyes, e.g. in colorimetric assays. Periodic acid can also be used to make sugars reactive towards crosslinkers, which can then be covalently bound to labeling molecules or an immobilized support (e.g., biotin, streptavidin) for purification and detection. Alternatively, glycans may be detected with glycan-specific antibodies or lectins. Lectins are carbohydrate-binding proteins that are highly specific for sugar moieties. Examples of lectins include concanavalin A (ConA), lentil lectin (LCH, snowdrop lectin (GNA), ricin (RCA), peanut agglutinin (PNA), jacalin (AIL), hairy vetch lectin (VVL), wheat germ agglutinin (WGA), elderberry lectin (SNA), maackia amurensis leukoagglutinin (MAL), Maackia amurensis hemoagglutinin (MAH), Ulex europaeus agglutinin (UEA), Aleuria aurantia lectin (AAL), and the like. Like antibodies, lectins can be conjugated to probes such as horseradish peroxidase, fluorophores, or biotin for visualization or immobilized to solid support, e.g. by interaction of these moieties to streptavidin or NeutrAvidin protein. Alternatively, glycans may be detected using Mass Spectrometry. For example, analyte of interest may be enriched using an analyte-specific antibody, the glycans released by enzymatic cleavage via endoglycanase H (endo H) or peptide-N4-(N-acetyl-beta-glucosaminyl)asparagine amidase (PNGase)), and the glycans further purified using liquid chromatography and quantified by tandem mass spectrometry. These and other exemplary methods for detecting and quantifying the amount of glycan on protein are well known in the art. See, e.g. Essentials of Glycobiology. 2nd edition. Varki A, Cummings RD, Esko JD, et al., editors. Cold Spring Harbor (NY): Cold Spring Harbor Laboratory Press 2009; Ruhaak et al. (2010) Glycan labeling strategies and their use in identification and quantification. Anal Bioanal Chem 397:3457-3481; and Roth et al. (2012) Identification and Quantification of Protein Glycosylation. Int. Journal of Carbohydrate Chem.

### METHODS

In some aspects of the invention, methods are provided for using the subject proteinuria biomarkers to provide a proteinuria assessment, e.g. a proteinuria diagnosis, proteinuria prognosis, monitoring proteinuria treatment, etc. In practicing the subject methods, a nonGly-suPAR analyte level for a biological sample (e.g., a blood sample or a urine sample) is evaluated to obtain a nonGly-suPAR analyte value for the sample. By a "nonGly-suPAR analyte value," it is meant a value that represents the level of nonGly-suPAR analyte in a biological sample from a subject. The terms "evaluating", "assaying", "measuring", "assessing," and "determining" are used interchangeably to refer to any form of measurement, including determining if an element is present or not, and including both quantitative and qualitative determinations. Evaluating may be relative or absolute.

By a "biological sample," it is meant any of a variety of sample types obtained from an organism which can be used in a diagnostic, prognostic, or monitoring assay, for example blood, urine, and other liquid samples of biological origin or cells derived therefrom and the progeny thereof. The term encompasses samples that have been manipulated in any way after their procurement, such as by treatment with reagents, solubilization, or enrichment for certain components. The term encompasses a clinical sample, and also includes cell supernatants, cell lysates, serum, plasma, biological fluids, and tissue samples. Clinical samples for use in the methods of the invention may be obtained from a variety of sources. In certain aspects, the "biological sample" or "sample from a subject" is a blood sample or a urine sample.

Sample sources of particular interest include blood samples or preparations thereof, e.g., whole blood, or serum or plasma, and urine. A sample volume of blood, serum, or urine between about 2µl to about 2,000µl is typically sufficient for determining the level of a nonGly-suPAR analyte, and hence a nonGly-suPAR analyte value. Generally, the sample volume will range from about 10µl to about 1,750µl, from about 20µl to about 1,500µl, from about 40µl to about 1,250µl, from about 60µl to about 1,000µl, from about 100µl to about 900 µl, from about 200µl to about 800µl, from about 400µl to about 600µl. In many embodiments, a suitable initial source for the human sample is a blood sample. In such instances, the sample employed in the subject assays is generally a blood-derived sample. The blood derived sample may be derived from whole blood or a fraction thereof, e.g., serum, plasma, etc., where in some embodiments the sample is derived from blood, allowed to clot, and the serum separated and collected to be used to assay.

In some embodiments the sample is a serum or serum-derived sample. Any convenient methodology for producing a fluid serum sample may be employed. In many embodiments, the method employs drawing venous blood by skin puncture (e.g., finger stick, venipuncture) into a clotting or serum separator tube, allowing the blood to clot, and centrifuging the serum away from the clotted blood. The serum is then collected and either stored or assayed.

Once a sample is obtained, it can be used directly, frozen, or maintained in appropriate culture medium for short periods of time until assayed to determine the nonGly-suPAR analyte value. Typically the samples will be from human patients, although animal models may find use, e.g. equine, bovine, porcine, canine, feline, rodent, e.g. mice, rats, hamster, primate, *etc*. Any convenient tissue sample that demonstrates the differential representation in a patient with proteinuria or at risk for developing proteinuria of the one or more suPAR analytes disclosed herein may be evaluated in the subject methods. Typically, a suitable sample source will be derived from fluids into which the molecular entity of interest, i.e. the RNA transcript or protein, has been released.

The subject sample may be treated in any of a variety of ways so as to enhance detection of analyte so as to obtain the nonGly-suPAR analyte value. For example, where the sample is blood, the red blood cells may be removed from the sample (e.g., by centrifugation, by lysis) prior to assaying. Such a treatment may serve to reduce any non-specific background levels associated with detecting the level of analyte using, e.g., an affinity reagent. Detection of analyte may also be enhanced by concentrating the sample using procedures well known in the art (e.g. acid precipitation, alcohol precipitation, salt precipitation, hydrophobic precipitation, filtration (using a filter which is capable of retaining molecules greater than 30 kD, e.g. Centrim 30™), affinity purification). In some embodiments, the pH of the test and control samples will be adjusted to, and maintained at, a pH which approximates neutrality (i.e. pH 6.5-8.0). Such a pH adjustment will prevent complex formation, thereby providing a more accurate quantitation of the level of marker in the sample. In embodiments where the sample is urine, the pH of the sample is adjusted and the sample is concentrated in order to enhance the detection of the marker.

Any convenient method for evaluating the level of nonGly-suPAR analyte in a biological sample from a subject may be employed in the subject methods. For example, the level of nonGly-suPAR analyte in a sample may be evaluated by directly detecting the amount of nonGly-suPAR polypeptide or variant thereof in the sample, e.g. using a nonGly-suPAR-specific antibody. As another example, the level of nonGly-suPAR analyte in a sample may be evaluated indirectly. For example, as discussed above, lectins have an affinity for glycan groups and may be used to distinguish glycosylated suPAR from non-glycosylated suPAR. As such, the sample may be contacted with lectin, e.g. bound to a solid support, e.g. a lectin-conjugated column, lectin-conjugated beads, etc. to remove glycosylated protein from the sample; and the protein that remains in the sample evaluated, e.g. using suPAR-specific antibodies, to determine the amount of nonGly-suPAR in the sample. As another example, suPAR analyte in the sample may be purified, e.g., by immunoprecipitation with a suPAR-specific antibody; and the amount of glycan in the purified protein measured, e.g. by mass spectrometry, by colorimetric assay (e.g. with periodic acid, with labeled lectin, etc.), and the like. Other methods will be readily understood by the ordinarily skilled artisan, e.g. as described in greater detail below.

As demonstrated by the examples above, in some instances, evaluating the level of nonGly-suPAR analyte in a biological sample may comprise detecting or purifying suPAR polypeptide or variant(s) or fragment(s) thereof in the biological sample. In some instances, e.g. as described above and in the working examples herein, evaluating the amount of nonGly-suPAR analyte may comprise detecting or purifying glycosylated proteins in a sample. Any convenient protocol and reagents for detecting or purifying suPAR protein or variants or fragments thereof and/or glycosylated proteins in the biological sample may be employed wherein the level of one or more proteins in the assayed sample is determined.

For example, detecting or purifying suPAR polypeptide or variant(s) or fragment(s) thereof in the biological sample may be performed with a suPAR analyte-specific affinity reagent. Similarly, detecting or purifying glycosylated polypeptide in the biological sample may be performed using a glycan-specific affinity reagent. By "affinity reagent" it is meant a reagent having an analyte binding domain, moiety or component that has a high binding affinity and binding specificity for the analyte (e.g. suPAR analyte, i.e. a suPAR polypeptide or a fragment or peptide thereof; or glycan moiety). By "high binding affinity" is meant a binding affinity of at least about 10⁻⁴ M, usually at least about 10⁻⁶ M or higher, e.g., 10⁻⁹ M or higher. The affinity reagent may be any of a variety of different types of molecules, so long as it exhibits the requisite binding affinity for the target protein when present as tagged affinity ligand. By "high binding specificity" and "binds specifically" is meant high avidity and/or high affinity binding of an affinity reagent to a specific antigen. For example, antibody binding to its epitope on this specific antigen is stronger than binding of the same antibody to any other epitope, particularly those which may be present in molecules in association with, or in the same sample, as the specific antigen of interest. Thus, for example, affinity reagents which bind specifically to a suPAR analyte of interest may be capable of binding other polypeptides at a weak, yet detectable, level (e.g., 10% or less of the binding shown to the polypeptide of interest). Such weak binding, or background binding, is readily discernible from the specific affinity reagent binding to the polypeptide of interest, e.g., by use of appropriate controls.

In some instances, the subject methods include the use of an affinity reagent having a high binding affinity and binding specificity for the suPAR analyte. In certain instances, the affinity reagent has a high binding affinity and binding specificity for nonGly-suPAR analyte, i.e., an epitope specific for the nonGly-suPAR polypeptide or fragment thereof, e.g., an epitope created by the absence of a glycan group on suPAR. In certain other instances, the affinity reagent has a high binding affinity and binding specificity for glycosylated suPAR analyte, i.e., an epitope specific for the glycosylated suPAR polypeptide or fragment thereof, e.g. an epitope created by the presence of a glycan group on suPAR.

According to certain embodiments, two or more affinity reagents are employed when practicing the methods, or may be included in the devices and kits, of the present disclosure. For example, aspects of the present disclosure include the use of: an affinity reagent (e.g., an antibody or binding fragment thereof) that binds to one or more of suPAR(I-III), suPAR(II-III), and suPAR(I), regardless of glycosylation status; and an affinity reagent (e.g., an antibody or binding fragment thereof) that only binds to the glycosylated form of one or more of suPAR(I-III), suPAR(II-III), and suPAR(I). Such a combination of antibodies finds use, e.g., in a diagnostic method, device, or kit in which the level of nonGly-suPAR is determined by measuring total suPAR protein levels (using an antibody that binds regardless of glycosylation status), and then subtracting the amount of glycosylated suPAR (measured using an antibody that only binds to glycosylated suPAR) to determine the amount of nonGly-suPAR present in a sample of interest. The antibody combination may be employed in any suitable assay format, including but not limited to, an ELISA-based assay format, a flow cytometric assay, or the like.

In some instances, because suPAR is a cleavage product of the uPAR protein, the suPAR analyte-specific affinity reagent may have high specificity for an epitope found in both suPAR and uPAR. In other words, in some instances, the suPAR analyte-specific affinity reagent will be specific for both suPAR and uPAR. In other instances, the suPAR analyte-specific affinity reagent will have specificity for only suPAR, e.g. it will be specific for an epitope found in suPAR and not uPAR, e.g., an epitope created by the cleavage of uPAR. In some instances, the subject methods include the use of an affinity reagent having a high binding affinity and binding specificity for glycosylated proteins in general, e.g. lectins.

An affinity reagent may be a small molecule ligand or large molecule ligand. By small molecule ligand is meant a ligand ranging in size from about 50 to about 10,000 daltons, usually from about 50 to about 5,000 daltons and more usually from about 100 to about 1000 daltons. By large molecule is meant a ligand ranging in size from about 10,000 daltons or greater in molecular weight.

The small molecule may be any molecule, as well as binding portion or fragment thereof, that is capable of binding with the requisite affinity and specificity to the target protein. Generally, the small molecule is a small organic molecule that is capable of binding to the target analyte of interest. The small molecule will include one or more functional groups necessary for structural interaction with the target analyte, e.g., groups necessary for hydrophobic, hydrophilic, electrostatic or even covalent interactions. Where the target analyte is a protein, e.g. a suPAR polypeptide, the small molecule will include functional groups necessary for structural interaction with proteins, such as hydrogen bonding, hydrophobic-hydrophobic interactions, electrostatic interactions, etc., and will typically include at least an amine, amide, sulfhydryl, carbonyl, hydroxyl or carboxyl group, preferably at least two of the functional chemical groups. The small molecule may also comprise a region that may be modified and/or participate in covalent linkage to a label component, a substrate surface, or other entity, depending on the particular assay protocol being employed, without substantially adversely affecting the small molecule's ability to bind to its target analyte.

Small molecule affinity ligands often comprise cyclical carbon or heterocyclic structures and/or aromatic or polyaromatic structures substituted with one or more of the above functional groups. Also of interest as small molecules are structures found among biomolecules, including peptides, saccharides, fatty acids, steroids, purines, pyrimidines, derivatives, structural analogs or combinations thereof. Such compounds may be screened to identify those of interest, where a variety of different screening protocols are known in the art.

The small molecule may be derived from a naturally occurring or synthetic compound that may be obtained from a wide variety of sources, including libraries of synthetic or natural compounds. For example, numerous means are available for random and directed synthesis of a wide variety of organic compounds and biomolecules, including the preparation of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known small molecules may be subjected to directed or random chemical modifications, such as acylation, alkylation, esterification, amidification, etc. to produce structural analogs.

As such, the small molecule may be obtained from a library of naturally occurring or synthetic molecules, including a library of compounds produced through combinatorial means, i.e. a compound diversity combinatorial library. When obtained from such libraries, the small molecule employed will have demonstrated some desirable affinity for the protein target in a convenient binding affinity assay. Combinatorial libraries, as well as methods for the production and screening, are known in the art and described in: 5,741,713; 5,734,018; 5,731,423; 5,721,099; 5,708,153; 5,698,673; 5,688,997; 5,688,696; 5,684,711; 5,641,862; 5,639,603; 5,593,853; 5,574,656; 5,571,698; 5,565,324; 5,549,974; 5,545,568; 5,541,061; 5,525,735; 5,463,564; 5,440,016; 5,438,119; 5,223,409.

Alternatively, the affinity reagent may be a large molecule. Of particular interest as large molecule affinity ligands are lectins. As discussed above, lectins are carbohydrate-binding proteins that are highly specific for sugar moieties. Examples of lectins include concanavalin A (ConA), lentil lectin (LCH, snowdrop lectin (GNA), ricin (RCA), peanut agglutinin (PNA), jacalin (AIL), hairy vetch lectin (VVL), wheat germ agglutinin (WGA), elderberry lectin (SNA), maackia amurensis leukoagglutinin (MAL), Maackia amurensis hemoagglutinin (MAH), Ulex europaeus agglutinin (UEA), Aleuria aurantia lectin (AAL), and the like. Also of particular interest as large molecule affinity ligands are antibodies, as well as binding fragments and mimetics thereof, with affinity and specificity for an antigenic fragment of suPAR. By "antigenic fragment" of suPAR is meant a portion of suPAR which is capable of binding an antibody generated by immunization of a mammal with suPAR or a fragment thereof. Preferably, the antibodies which specifically bind an epitope of the isolated antigenic fragment will also bind the same epitope in the context of the native protein from which the fragment was derived. Examples of antibodies with specificity for human suPAR analyte known in the art include the antibodies ATN615 (Li et al. An anti-urokinase plasminogen activator receptor (uPAR) antibody: crystal structure and binding epitope. J. Mol. Biol. 2007 Jan 26;365(4):1117-29); IIIF10 and HD13.1 (Kotzsch et al. New ELISA for quantitation of human urokinase receptor (CD87) in cancer. Int. J. Oncol. 2000 Oct;17(4):827-34); R2, R3, R5, R9, and R23 (Piironen et al. Specific immunoassays for detection of intact and cleaved forms of the urokinase receptor. Clin. Chem. 2004;50:2059-68); and the antibodies disclosed in Haastrup et al. (Soluble urokinase plasminogen activator receptor during allogeneic stem cell transplantation. Scand. J. Immunol. 2011 Apr;73(4):325-9), Lönnkvist et al. (Blood chemistry markers for evaluation of inflammatory activity in Crohn's disease during infliximab therapy. Scand. J. Gastroenterol. 2011 Apr;46(4):420-7), Gao et al. (Detection of soluble urokinase receptor by immunoradiometric assay and its application in tumor patients. Thromb. Res. 2001 Apr 1;102(1):25-31) and PCT Publication No. WO 2010/054189. Also suitable for use as large molecule affinity ligands are polynucleic acid aptamers. Polynucleic acid aptamers may be RNA oligonucleotides which may act to selectively bind proteins, much in the same manner as a receptor or antibody (Conrad et al., Methods Enzymol. (1996), 267(Combinatorial Chemistry), 336-367).

Where antibodies are the affinity ligand, they may be a polyclonal composition, i.e. a heterogeneous population of antibodies differing by specificity. Alternatively, they may be monoclonal compositions, i.e. a homogeneous population of identical antibodies that have the same specificity for the target protein. As such, the affinity ligand may be either a monoclonal and polyclonal antibody. In yet other embodiments, the affinity ligand is an antibody binding fragment or mimetic, where these fragments and mimetics have the requisite binding affinity for the target protein. For example, antibody fragments, such as Fv, F(ab)2, Fab' and Fab may be prepared by cleavage of the intact protein, e.g. by protease or chemical cleavage. Also of interest are recombinantly produced antibody fragments, such as single chain antibodies or scFvs, where such recombinantly produced antibody fragments retain the binding characteristics of the above antibodies. Such recombinantly produced antibody fragments generally include at least the VH and VL domains of the subject antibodies, so as to retain the binding characteristics of the subject antibodies. These recombinantly produced antibody fragments or mimetics of the subject invention may be readily prepared using any convenient methodology, such as the methodology disclosed in U.S. Patent Nos. 5,851,829 and 5,965,371.

The above described lectins and antibodies, fragments and mimetics thereof may be obtained from commercial sources and/or prepared using any convenient technology, where methods of producing polyclonal antibodies, monoclonal antibodies, fragments and mimetics thereof, including recombinant derivatives thereof, are known to those of the skill in the art.

In some instances, the affinity reagent may be detectably labeled, e.g. to facilitate detection. By "detectably labeled affinity reagent" and "detectably labeled antibody" it is meant an affinity reagent, e.g., antibody (or antibody fragment which retains binding specificity), lectin, etc. having an attached detectable label. The detectable label may be attached by chemical conjugation, but where the label is a polypeptide, it could alternatively be attached by genetic engineering techniques. Methods for production of detectably labeled proteins are well known in the art. Detectable labels may be selected from a variety of such labels known in the art, but normally are radioisotopes, fluorophores, enzymes (e.g., horseradish peroxidase), or other moieties or compounds which either emit a detectable signal (e.g., radioactivity, fluorescence, color) or emit a detectable signal after exposure of the label to its substrate. Various detectable label/substrate pairs (e.g., horseradish peroxidase/diaminobenzidine, avidin/streptavidin, luciferase/luciferin), methods for labeling antibodies and lectins, and methods for using labeled antibodies to detect antigens (e.g. suPAR or suPAR fragments) and lectins to detect glycans are well known in the art (see, for example, Harlow and Lane, eds. Antibodies: A Laboratory Manual (1988) Cold Spring Harbor Laboratory Press, Cold Spring Harbor, N.Y.; Essentials of Glycobiology. 2nd edition. Varki A, Cummings RD, Esko JD, et al., editors. Cold Spring Harbor (NY): Cold Spring Harbor Laboratory Press 2009; Ruhaak et al. (2010) Glycan labeling strategies and their use in identification and quantification. Anal Bioanal Chem 397:3457-3481; and Roth et al. (2012) Identification and Quantification of Protein Glycosylation. Int. Journal of Carbohydrate Chem).

Any convenient assay protocol may be employed. For example, the assay may be performed in solution. As another example, the assay may be performed on a solid (insoluble) support (e.g. polystyrene, nitrocellulose, beads, etc.). Examples of assay formats include ELISAs (enzyme-linked immunosorbent assays; see, for example, the SUPARNOSTIC® ELISA kit (ViroGates), Kotzsch et al. Int. J. Oncol. 2000 Oct;17(4):827-34, and Ronne et al. J. Immunol. Methods. 1994 Jan 3;167(1-2):91-101 for ELISAs for the detection of suPAR, and Piironen et al. Clin. Chem. 2004;50:2059-68 and Henic et al. Clin. Cancer Res. 2008 Sep 15;14(18):5785-93 for time-resolved fluorescence assays for the detection of suPAR(I-III), suPAR(II-III), suPAR(I-III) + suPAR(II-III), and uPAR(I)); IRMAs (immunoradiometric assays; see, for example, Gao et al. Thromb Res. 2001 Apr 1;102(1):25-31 for immunoradiometric assays to detect suPAR); and RIAs (radioimmunoassays), using any standard methods (e.g., as described in Current Protocols in Immunology, Coligan et al., ed.; John Wiley & Sons, New York, 1992). Typically, the assay will be performed in the presence of a control, e.g. a positive control or a negative control. For example, in certain embodiments, a series of standards containing known concentrations of suPAR may be assayed in parallel with the samples or aliquots thereof to serve as positive controls. Furthermore, in certain embodiments, each sample and standard will be added to multiple wells so that mean values can be obtained for each.

For example, where the assay is performed in solution, the test and control samples may each be incubated with a suPAR analyte affinity reagent for a time period sufficient to allow formation of analyte and affinity reagent complexes in solution, preferably between about 1 minute up to 24 hrs, or more. As previously noted, the affinity reagent may include a detectable label (e.g. radionuclide, fluorescer, or enzyme). The sample may then be treated to separate the analyte and affinity reagent complexes from excess, unreacted affinity reagent, for example by addition of an anti-affinity reagent composition (e.g., anti-immunoglobulin antiserum) followed by centrifugation (e.g., 1000xg for 7 min) to precipitate the analyte and affinity reagent complexes, or by binding to an affinity surface such as a second, unlabelled suPAR analyte affinity reagent (e.g., antibody) fixed to a solid substrate such as Sepharose or a plastic well. Detection of affinity reagent bound to a suPAR analyte may be achieved in a variety of ways well known in the art. If necessary, a substrate for the detectable label may be added to the sample.

As another example, where the assay uses a solid support, the support may have an affinity reagent (or combination of two or more affinity reagents) capable of specifically binding suPAR analyte or glycans, where the affinity reagent is bound to the support surface. The affinity reagent facilitates the stable, wash-resistant binding of a suPAR analyte present in the sample to the solid support. The insoluble supports may be any compositions to which affinity reagents, such as antibodies or fragments and mimetics thereof can be bound, which is readily separated from soluble material, and which is otherwise compatible with the overall method of measuring a suPAR analyte in the sample. The surface of such supports may be solid or porous and of any convenient shape. Examples of suitable insoluble supports to which the affinity reagent is bound include beads, membranes, and microtiter plates. These are typically made of glass, plastic (e.g. polystyrene), polysaccharides, nylon or nitrocellulose. Microtiter plates are especially convenient because a large number of assays can be carried out simultaneously, using small amounts of reagents and samples. Methods for binding affinity reagents (e.g., antibodies, or fragments and mimetics thereof) to solid supports are well known in the art. After binding of the affinity reagent to the support, the support may be treated with a blocking agent, which binds to the support in areas not occupied by the affinity reagent. Suitable blocking agents include non-interfering proteins such as bovine serum albumin, casein, gelatin, and the like. Alternatively, several detergents at non-interfering concentrations, such as Tween, NP40, TX100, and the like may be used. Such blocking treatment reduces nonspecific binding. Alternatively, the solid support itself may bind a suPAR analyte directly through the charged properties of the support surface, thus taking advantage of the charged nature of a suPAR analyte molecule. Similarly, periodic acid may be employed to crosslink proteins comprising glycans to a solid support.

The test and control samples (if used) are each incubated with the solid support for a time sufficient for binding of analyte, e.g. suPAR analyte, total glycosylated protein, etc. to the affinity reagent. Generally from about 0.001 to 1 ml of sample, diluted or otherwise, is sufficient, usually about 0.01 ml sufficing. The incubation time should be sufficient for analyte, e.g. suPAR protein, glycosylated protein, etc. to bind the insoluble first affinity reagent. Generally, from about 0.1 to 3 hr is sufficient, usually 1 hr sufficing. After incubation, the reacted samples may be washed to remove unbound or non-specifically bound material. Generally, a dilute non-ionic detergent medium at an appropriate pH, generally 7-8, may be used as a wash medium. An isotonic buffer, such as phosphate-buffered saline, may be employed in the washing step. From one to six washes may be employed, with sufficient volume to thoroughly wash non-specifically bound proteins present in the sample. Preferably, the washing step will not cause dissociation of analyte/affinity reagent complexes.

A second affinity reagent which specifically binds analyte, e.g. an anti-suPAR analyte antibody, or fragment or mimetic thereof which preferably binds to a suPAR epitope different from the epitope bound by the first affinity reagent, is then incubated with the suPAR analyte-affinity reagent complexes. The concentration of the second affinity reagent will generally be about 0.1 to 50 µg/ml, preferably about 1 µg/ml. The solution containing the second antibody is generally buffered in the range of about pH 6.5-9.5. The incubation time should be sufficient for the second affinity reagent to bind available molecules. Generally, from about 0.1 to 3 hr is sufficient, usually 1 hr sufficing. After the second affinity reagent has bound, the insoluble support is generally again washed free of non-specifically bound second receptor, essentially as described for prior washes. After non-specifically bound material has been cleared, the signal produced by the bound conjugate is detected by conventional means.

The bound conjugate may be detected by any convenient method. For example, the second affinity reagent used to detect suPAR analyte bound to the support may be detectably labeled to facilitate direct or indirect detection of suPAR analyte-first affinity reagent-second affinity reagent complexes. Examples of labels which permit direct measurement of immunocomplexes include radiolabels, such as ³H or ¹²⁵I, fluorescers, dyes, beads, chemilumninescers, colloidal particles, and the like. Examples of labels which permit indirect measurement of binding include enzymes where the substrate may provide for a colored or fluorescent product. In some embodiment, the second affinity reagent (e.g., antibody or fragment and mimetic thereof) is labeled with a covalently bound enzyme capable of providing a detectable product signal after addition of suitable substrate. Examples of suitable enzymes for use in conjugates include horseradish peroxidase, alkaline phosphatase, malate dehydrogenase and the like. Where not commercially available, such affinity reagent-enzyme conjugates are readily produced by techniques known to those skilled in the art.

Alternatively, a third detectably labeled affinity reagent (e.g., antibody, or fragment and mimetic thereof) which specifically binds the second affinity reagent may be used to detect the suPAR analyte-first affinity reagent-second affinity reagent complexes. Examples of third affinity reagent/second affinity reagent-specific molecule pairs include antibody/anti-antibody and avidin (or streptavidin)/biotin. Since the resultant signal is thus amplified, this technique may be advantageous where only a small amount of a suPAR analyte is present in the sample. An example is the use of a labeled antibody specific to the second antibody. The volume, composition and concentration of the third affinity reagent solution provides for measurable binding to the suPAR analyte already bound to the second affinity reagent. Generally, the same volume as that of the sample is used: from about 0.001 to 1 ml is sufficient, usually about 0.1 ml sufficing. The concentration will generally be sufficient to saturate the suPAR analyte potentially bound to second reagent.

Where an enzyme conjugate is used for detection, an appropriate enzyme substrate is provided so a detectable product is formed. More specifically, where a peroxidase is the selected enzyme conjugate, a preferred substrate combination is H₂O and O-phenylenediamine which yields a colored product under appropriate reaction conditions. Appropriate substrates for other enzyme conjugates such as those disclosed above are known to those skilled in the art. Suitable reaction conditions as well as means for detecting the various useful conjugates or their products are also known to those skilled in the art. For the product of the substrate O-phenylenediamine for example, light absorbance at 490-495 nm is conveniently measured with a spectrophotometer.

As another example of an assay format, analyte, e.g. suPAR analyte, may be detected by using a competitive binding assay. The test and control samples are incubated with the affinity reagent, e.g. as described above, to allow for formation of analyte/affinity reagent complexes. The affinity reagent may be fixed to a solid surface or in solution. After washing to remove unbound material from the precipitated suPAR analyte/affinity reagent complexes or from the solid support (if any) to which the affinity reagent is fixed, the samples are then incubated with a standard amount of competitive suPAR, e.g. competitive recombinant hybrid suPAR or competitive suPAR fragment which retains the ability to compete with a native suPAR analyte, for binding to the anti-suPAR analyte affinity reagent. In some instances, the competitive suPAR reagent may be detectably labeled to facilitate detection. In other words, detectably labeled suPAR, detectably labeled recombinant hybrid suPAR, or a detectably labeled fragment of suPAR may be used. By "detectably labeled suPAR", "detectably labeled recombinant hybrid suPAR" and "detectably labeled suPAR fragment" is meant a suPAR polypeptide or suPAR polypeptide/peptide fragment having an attached detectable label, e.g. as described above for detectably labeled affinity reagents. Binding is detected by standard means: e.g., by measuring the amount of label associated with (a) the solid support (if any), or (b) the precipitated analyte/binding agent complexes. In other instances, the competitive suPAR (i.e. the suPAR introduced into the test sample after incubation of the test sample with the anti- suPAR analyte affinity reagent) may be labeled with an epitope that is absent from the suPAR analyte derived from the sample of body fluid, and the detection of the binding of competitive suPAR molecule facilitated by detecting the epitope. For example, the competitive suPAR molecule may be a recombinant fusion protein which retains the ability to bind competitively to the affinity reagent used in the assay. Binding of suPAR fusion protein to the anti-suPAR affinity reagent may then be detected by incubating the sample with a detectably labeled second affinity reagent which specifically binds the fusion protein and does not bind the suPAR analyte from the sample. An example of a recombinant suPAR fusion protein is one that contains an N-terminal extension of amino acids, which recombinant suPAR fusion protein may be used in such a detection method, since affinity reagents which specifically bind to the N-terminal amino acid extension of the recombinant molecule would not be expected to bind to a suPAR analyte present in a sample. Examples of other epitopes which may be introduced into a suPAR fusion protein include epitopes for use as targets for chemical modification and epitopes which have an altered amino acid sequence relative to a naturally-occurring suPAR analyte (to provide a peptide epitope absent in a suPAR analyte). A lower level of binding of the detectably labeled suPAR in the test sample than in the negative control, e.g. a sample comprising a level of suPAR analyte comparable to that found in a healthy individual, indicates the presence of an elevated level of suPAR analyte in the test sample.

In some instances, more than one suPAR analyte-specific affinity reagents may be employed. For example, in some instances, the suPAR analyte-specific affinity reagent will have specificity for an epitope found in both suPAR and uPAR, since suPAR is derived from uPAR. In other words, the suPAR analyte-specific affinity reagent will actually be specific for uPAR as well as suPAR. In some instances, it may be desirable to distinguish between suPAR and uPAR by, for example, using a first suPAR analyte-specific affinity reagent that is specific for suPAR (i.e. that detects both suPAR and uPAR) and a second affinity reagent that is specific only for uPAR (e.g. that detects the GPI anchor of uPAR).

As another example, it may be desirable to employ suPAR analyte-specific affinity reagents that are capable of distinguishing between the various suPAR analyte variants, e.g. suPAR(I-III), suPAR(II-III), and suPAR(I), or glycosylated suPAR versus non-glycosylated suPAR. For example, in some embodiments a single type of affinity reagent that recognizes all variants of suPAR may be employed. However, in other embodiments it may be desirable to use different affinity reagents that recognize specific variants of suPAR, e.g. total suPAR and nonGly-suPAR, or total suPAR and glycosylated suPAR. As such, in some embodiments, the subject assay of the present invention will detect the level of only one variant of suPAR in a sample. In other embodiments, the subject assay of the present invention will detect the level of more than one variants of suPAR in the sample.

In certain aspects, two or more affinity reagents are employed when practicing the subject methods. For example, aspects of the present disclosure include the use of: an affinity reagent (e.g., an antibody or binding fragment thereof) that binds to one or more of suPAR(I-III), suPAR(II-III), and suPAR(I), regardless of glycosylation status; and an affinity reagent (e.g., an antibody or binding fragment thereof) that only binds to the glycosylated form of one or more of suPAR(I-III), suPAR(II-III), and suPAR(I). Such a combination of antibodies finds use, e.g., in a diagnostic method, device, or kit in which the level of nonGly-suPAR is determined by measuring total suPAR protein levels (using an affinity reagent that binds regardless of glycosylation status), and then subtracting the amount of glycosylated suPAR (measured using an antibody that only binds to glycosylated suPAR) to determine the amount of nonGly-suPAR present in a sample of interest. The antibody combination may be employed in any suitable assay format, such as an ELISA-based assay format, a flow cytometric assay, or any other convenient assay format.

One representative and convenient type of protocol for assaying protein levels is ELISA. In ELISA and ELISA-based assays, one or more antibodies specific for the proteins of interest may be immobilized onto a selected solid surface, preferably a surface exhibiting a protein affinity such as the wells of a polystyrene microtiter plate. After washing to remove incompletely adsorbed material, the assay plate wells are coated with a non-specific "blocking" protein that is known to be antigenically neutral with regard to the test sample such as bovine serum albumin (BSA), casein or solutions of powdered milk. This allows for blocking of non-specific adsorption sites on the immobilizing surface, thereby reducing the background caused by non-specific binding of antigen onto the surface. After washing to remove unbound blocking protein, the immobilizing surface is contacted with the sample to be tested under conditions that are conducive to immune complex (antigen/antibody) formation. Such conditions include diluting the sample with diluents such as BSA or bovine gamma globulin (BGG) in phosphate buffered saline (PBS)/Tween or PBS/Triton-X 100, which also tend to assist in the reduction of nonspecific background, and allowing the sample to incubate for about 2-4 hours at temperatures on the order of about 25°-27°C (although other temperatures may be used). Following incubation, the antisera-contacted surface is washed so as to remove non-immunocomplexed material. An exemplary washing procedure includes washing with a solution such as PBS/Tween, PBS/Triton-X 100, or borate buffer. The occurrence and amount of immunocomplex formation may then be determined by subjecting the bound immunocomplexes to a second antibody having specificity for the target that differs from the first antibody and detecting binding of the second antibody. In certain embodiments, the second antibody will have an associated enzyme, e.g. urease, peroxidase, or alkaline phosphatase, which will generate a color precipitate upon incubating with an appropriate chromogenic substrate. For example, a urease or peroxidase-conjugated anti-human IgG may be employed, for a period of time and under conditions which favor the development of immunocomplex formation (e.g., incubation for 2 hr at room temperature in a PBS-containing solution such as PBS/Tween). After such incubation with the second antibody and washing to remove unbound material, the amount of label is quantified, for example by incubation with a chromogenic substrate such as urea and bromocresol purple in the case of a urease label or 2,2'-azino-di-(3-ethyl-benzthiazoline)-6-sulfonic acid (ABTS) and H₂O₂, in the case of a peroxidase label. Quantitation is then achieved by measuring the degree of color generation, e.g., using a visible spectrum spectrophotometer.

The preceding format may be altered by first binding the sample to the assay plate. Then, primary antibody is incubated with the assay plate, followed by detecting of bound primary antibody using a labeled second antibody with specificity for the primary antibody.

The solid substrate upon which the antibody or antibodies are immobilized can be made of a wide variety of materials and in a wide variety of shapes, e.g., microtiter plate, microbead, dipstick, resin particle, etc. The substrate may be chosen to maximize signal to noise ratios, to minimize background binding, as well as for ease of separation and cost. Washes may be effected in a manner most appropriate for the substrate being used, for example, by removing a bead or dipstick from a reservoir, emptying or diluting a reservoir such as a microtiter plate well, or rinsing a bead, particle, chromatographic column or filter with a wash solution or solvent.

Alternative, non-ELISA based-formats for measuring the levels of protein or glycosylated protein in a sample may be employed. Representative examples include but are not limited to mass spectrometry, proteomic arrays, xMAP™ microsphere technology, flow cytometry, western blotting, and immunohistochemistry. For example, glycosylated proteins may be detected with periodic acid, which makes glycans reactive with dyes. Periodic acid may also be employed to crosslink proteins comprising glycans to a solid support.

The measured levels of nonGly-suPAR analyte so obtained may be used as the nonGly-suPAR analyte value for the sample. Alternatively, the measured levels may be analyzed in any of a number of ways to obtain a nonGly-suPAR analyte value. For example, the level of nonGly-suPAR analyte may be normalized, e.g. relative to the expression of a selected housekeeping gene, e.g. ABL1, GAPDH, or PGK1. As another example, the level of nonGly-suPAR analyte may be compared to the level of total suPAR or glycosylated suPAR, to arrive at a ratio. As another example, the level of nonGly-suPAR analyte may be considered with other proteinuria markers known in the art, e.g. to arrive at a proteinuria score.

In some instances, the subject methods of determining or obtaining a nonGly-suPAR analyte value for a sample for a subject further comprise providing the nonGly-suPAR analyte value as a report. Thus, in some instances, the subject methods may further include a step of generating or outputting a report providing the results of a nonGly-suPAR analyte evaluation in the sample, which report can be provided in the form of an electronic medium (e.g., an electronic display on a computer monitor), or in the form of a tangible medium (e.g., a report printed on paper or other tangible medium). Any form of report may be provided, e.g. as known in the art or as described in greater detail below.

### UTILITY

The compositions and methods of the present disclosure find use in a variety of different applications (including research and/or clinical (e.g., clinical diagnostic) applications), in which it is desirable, e.g., to determine a nonGly-suPAR analyte value in a sample of interest. In some aspects of the subject methods, the nonGly-suPAR analyte value is employed to provide a proteinuria assessment. By a proteinuria assessment, it is meant a proteinuria diagnosis, proteinuria prognosis, the results of monitoring the proteinuria, suggestions for treating the proteinuria, and the like. The term "proteinuria" is used herein to mean the presence of excessive amounts of serum protein in the urine. A number of methods are known in the art for detecting proteinuria including, for example, a quantitative protein determination in a timed (24 hour) urine collection, a quantitative protein determination of the ratio of protein levels to creatinine levels (the protein/creatinine ratio, or "PCR") in a spot urine collection, a foamy appearance or excessive frothing of the urine, etc. Such methods will be understood by the ordinarily skilled artisan. Typically, albumin levels are detected, with substantially normal levels of albumin in the urine ("normoalbuminuria") being about 30mg or less in a 24 hour collection (30mg or less/day), or an ACR of about 30 µg or less albumin / mg creatinine ("30µg or less/mg"); modestly elevated levels of albumin ("microalbuminuria") being about 30 to 300 mg in a 24 hour urine collection (30-300mg/24 hours) or an ACR of about 30 to 300 µg albumin /mg creatinine ("30-300µg/mg"); and significantly high levels of albumin ("macroalbuminuria") being about 300mg or more in a 24 hour urine collection ("more than 300mg/24 hours"), or as an ACR of 300 µg albumin or more per mg creatinine ("300µg or more/mg").

Proteinuria is often a symptom of renal (kidney) distress, urinary distress, pancreatic distress, nephrotic syndromes (i.e., proteinuria larger than 3.5 grams per day), glomerular diseases such as membranous nephropathy (MN) and focal segmental glomerulosclerosis (FSGS), eclampsia, and toxic lesions of kidneys, and may be a symptom of diabetes, e.g. diabetic kidney disease (diabetic nephropathy (DNP)) and of cardiovascular disease. With severe proteinuria, general hypoproteinemia may develop, resulting in diminished oncotic pressure (ascites, edema, hydrothorax).

One example of a disease or disorder that may be associated with proteinuria is diabetes. Diabetes is a metabolic disease that occurs when the pancreas does not produce enough of the hormone insulin to regulate blood sugar ("type 1 diabetes mellitus") or, alternatively, when the body cannot effectively use the insulin it produces ("type 2 diabetes mellitus"). Type 1 diabetes, also known as insulin dependent diabetes mellitus (IDDM), results from the destruction or dysfunction of β cells by the cells of the immune system. Symptoms include polyuria (frequent urination), polydipsia (increased thirst), polyphagia (increased hunger), and weight loss. T1D is fatal unless treated with insulin and must be continued indefinitely, although many people who develop the disease are otherwise healthy and treatment need not significantly impair normal activities. Type 2 diabetes, also known as non-insulin dependent diabetes mellitus (NIDDM), is associated with resistance to insulin in peripheral tissues (such as skeletal muscles and liver) and by a gradual decline in β cell function and numbers over time, as the β cells develop resistance to insulin as well. As a result, in T2D the pancreas does not make enough insulin to keep blood glucose levels normal. Symptoms include hyperglycemia (high blood sugar), diabetic ketoacidosis (increased ketones in urine), and hyperosmolar hyperglycemic non-ketotic syndrome.

In some instances, diabetes may progress to diabetic kidney disease. By a "diabetic kidney disease" or "diabetic nephropathy" it is meant a chronic condition caused by or associated with diabetes in which the function of the kidneys in removing waste products and excess fluid from the body declines slowly and progressively. Symptoms of diabetic kidney disease include the occurrence of microalbuminuria or macroalbuminuria, or the progressive decline of GFR in a normoalbuminuric individual with any form of diabetes. In early stage diabetic kidney disease, the subject may present with normoalbuminuria or microalbuminuria and normal or high GFR, i.e. a GFR of 90 mL/min/1.73 m² or more. In progressive diabetic kidney disease the proteinuria will have advanced to macroalbuminuria or stage 2 chronic kidney disease (CKD) or worse, i.e., a glomerular filtration rate (GFR) of less than 90 cc/min/1.73m².

Another example of a disorder associated with proteinuria is nephritis. Nephritis is an inflammation of the kidneys. Evidence, e.g., blood and/or protein in the urine and impaired kidney function, etc., of nephritis depends on the type, location, and intensity of the immune response, inflammation affecting the glomeruli, the tubules, the tissue around the tubules, or blood vessels. "Nephritis-related disease" include, but are not limited to, e.g., primary glomerulopathies (acute diffuse proliferative glomerulonephritis, post-streptococcal glomerulopathy, non-post streptococcal glomerulopathy, crescentic glomerulonephritis, membraneous glomerulopathy, lipoid nephrosis, focal segmental glomerulosclerosis, membranoproliferative glomerulonephritis, IgA nephropathy, focal proliferative glomerulonephritis, and chronic glomerulonephritis), systemic diseases (systemic lupus erythematosus, diabetes mellitus, amyloidosis, Goodpasture's syndrome, polyarteritis nodosa, Welgener's granulomatosis, Henoch-Schonlein purpura, and Bacterial endocarditis), and hereditary disorders (Alport's syndrome, thin membrane disease, and Fabry's disease).

Another example of a disease or disorder associated with proteinuria is glomerulosclerosis. Glomerulosclerosis is a degenerative kidney disease that results in hyaline deposits or scarring within the renal glomeruli often associated with renal arteriosclerosis or diabetes. Typically, there is an infiltration of circulating inflammatory cells, fibrosis of interstitium and tubular atrophy. Glomerular injury caused by several factors brings about proteinuria in which proteins bind with soluble immunoglobulin A (slgA), slgG and slgM, forming immune complexes on the basement membrane. These immune complexes function as a chemotactic factor for inflammatory lymphocytes, which cause excessive immune responses in the affected areas (Bohle A. et al., Kidney Int. 67 (Suppl.): 186S-188S (1998)). When tubules are damaged by inflammatory cells, blood vessels connected with glomeruli are also injured and occluded. As a consequence, glomeruli become adversely affected and deteriorate. These glomerular changes are accompanied by tissue fibrosis and progress into eventual renal failure (see, e.g., Ratscchek M et al., Clin. Nephrol. 25: 221-226 (1986); Bohle A. et al, Clin. Nephrol. 29: 28-34 (1998); Bohle A et al., Kidney Blood Press Res. 19:191-195 (1996)). Of particular interest is Focal Segmental Glomerulosclerosis (FSGS). FSGS is a segmental collapse of the glomerular capillaries with thickened basement membranes and increased mesangial matrix, which often results in proteinuria and renal insufficiency. See, e.g., Kamanna et al., Histol. Histopathol. 13: 169-179(1998); Wehrmann et al., Clin. Nephrol. 33:115-122 (1990); Mackensen-Haen, et al., Clin. Nephrol. 37:70-77 (1992). FSGS is a cause of nephrotic syndrome in children and adolescents, characterized by edema (associated with weight gain), hypoalbuminemia (low serum albumin, a protein in the blood), hyperlipidemia, and hypertension (high blood pressure); and of proteinuria and kidney failure in adults. It can cause permanent kidney failure.

Other examples of diseases associated with proteinuria will be readily known to the ordinarily skilled artisan, any of which may be assessed using the subject methods and compositions. For example, proteinuria is typically associated with increased serum suPAR levels. As such, pathological conditions in which elevated serum suPAR levels are detected may be assessed for proteinuria using the subject methods, for example, paroxysmal nocturnal hemoglobinuria, HIV-1 infection, pneumococcal bacteremia, malaria, sepsis, bacterial and viral CNS infection, TB, and also various forms of solid tumors (breast, prostate, and ovarian cancer) (See, e.g., Montuori, N., and Ragno, P. (2009). Multiple activities of a multifaceted receptor: roles of cleaved and soluble uPAR. Frontiers in bioscience : a journal and virtual library 14, 2494-2503; and Thuno, M., et al. (2009). suPAR: the molecular crystal ball. Disease markers 27, 157-172).

In some instances, the nonGly-suPAR analyte value is employed to diagnose proteinuria; that is, to provide a determination as to whether a subject is affected by (e.g., exhibits) proteinuria, the severity of proteinuria, etc. In some instances, the subject may present with clinical symptoms of proteinuria, e.g., macroalbuminuria, a low glomerular filtration rate of e.g. less than about 90 cc/min/1.73m², etc. By "glomerular filtration rate" or GFR it is meant the flow rate of filtered fluid through the kidney. In other words, it is the volume of fluid filtered from the renal (kidney) glomerular capillaries into the Bowman's capsule per unit time. GFR may be determined by a number of different techniques. For example, inulin or the inulin-analog sinistrin may be injected into the plasma and its excretion in urine measured. As another example, GFR may be approximated based on determined (CCr) or estimated (eCCr) rate of creatinine clearance from the body using any convenient methodology. In other instances, subject may be asymptomatic for proteinuria by traditional clinical methods, e.g. normalbuminuric with a GFR of 90 mL/min/1.73 m² or more, but has risk factors associated with proteinuria, e.g. a medical condition such as diabetes, FSGS, microalbuminuria, hypertension, or preeclampsia, heritage (African American, American Indian, Hispanic American, Pacific Islander American), older age, obesity, or a family history of kidney disease. In yet other instances, the subject may be asymptomatic for proteinuria by traditional clinical methods and have no risk factors associated with proteinuria.

In some instances, the nonGly-suPAR analyte value is employed to prognose proteinuria; that is, to provide a proteinuria prognosis. For example, the nonGly-suPAR analyte value of a biological sample from a subject that does not have proteinuria may be used to predict a subject's susceptibility, or risk, of developing proteinuria. By predicting if the individual will develop proteinuria, it is meant determining the likelihood that a subject will develop proteinuria in the next week, in the next 2 weeks, in the next 3 weeks, in the next 4 weeks, in the next 2 months, in the next 3 months, in the next 6 months or more. As another example, the nonGly-suPAR analyte value of a biological sample from a subject that has proteinuria may be used to predict the course of disease progression and/or disease outcome, e.g. expectations as to whether an early stage kidney disease, e.g. early stage diabetic kidney disease, will develop into progressive diabetic kidney disease, e.g. stage 2 chronic kidney disease or worse.

In some instances, the nonGly-suPAR analyte value is employed to predict a subject's responsiveness to treatment for the proteinuria, e.g., positive response, a negative response, no response at all. In some instances, the nonGly-suPAR analyte value is employed to monitor a proteinuria. By "monitoring" a proteinuria, it is generally meant monitoring a subject's condition, e.g. to inform a proteinuria diagnosis, to inform a proteinuria prognosis, to provide information as to the effect or efficacy of a proteinuria treatment, and the like.

In some instances, the nonGly-suPAR analyte value may be employed to determine a treatment for a subject. The terms "treatment", "treating" and the like are used herein to generally mean obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment" as used herein covers any treatment of a proteinuria in a mammal, and includes: (a) preventing the proteinuria from occurring in a subject which may be predisposed to the proteinuria but has not yet been diagnosed as having it; (b) inhibiting the proteinuria, i.e., arresting its development; or (c) relieving the proteinuria, i.e., causing regression of the proteinuria. The therapeutic agent may be administered before, during or after the onset of proteinuria. The treatment of ongoing proteinuria, where the treatment stabilizes or reduces the undesirable clinical symptoms of the patient, is of particular interest. The subject therapy may be administered prior to the symptomatic stage of the proteinuria, and in some cases after the symptomatic stage of the disease. The terms "individual," "subject," "host," and "patient," are used interchangeably herein and refer to any mammalian subject for whom diagnosis, treatment, or therapy is desired, particularly humans. Proteinuria treatments may include any treatment known in the art, including, for example, ACE inhibitors (angiotensin-converting enzyme inhibitors), ARBs (angiotensin receptor blockers), and the like.

In some embodiments, the subject methods of providing a proteinuria assessment, e.g. diagnosing proteinuria, prognosing proteinuria, monitoring the proteinuria, and the like, may comprise comparing the obtained nonGly-suPAR analyte value to a proteinuria reference element to identify similarities or differences with the reference element, where the similarities or differences that are identified are then employed to provide the proteinuria assessment, e.g. diagnose the proteinuria, prognose the proteinuria, monitor the proteinuria, determine a proteinuria treatment, etc. By a "proteinuria reference element" it is meant an element, e.g. a tissue sample, a proteinuria value, a range of values, and the like that is representative of a phenotype (in this instance, a proteinuria phenotype) and may be used to determine the phenotype of the subject, e.g. if the subject is healthy or is affected by proteinuria, if the subject is at risk for developing proteinuria, if the subject has proteinuria that is responsive to therapy, etc.

For example, a proteinuria reference element may be a sample from an individual that has or does not have proteinuria, which may be used, for example, as a reference/control in the experimental determination of the marker value for a given subject. As another example, a proteinuria reference element may be a nonGly-suPAR analyte value or range of values which is representative of a proteinuria state and may be used as a reference/control to interpret the nonGly-suPAR analyte value of a given subject. For example amount of nonGly-suPAR in a sample is typically at least 1.5-fold or greater, e.g. 2-fold, 2.5-fold, 3-fold, 4-fold, 5-fold, 6-fold, 7-fold-old, 8-fold, 9-fold, 10-fold, or greater in a sample associated with the proteinuria phenotype than in a sample not associated with the proteinuria phenotype. The proteinuria reference element may be a positive reference/control, e.g., a sample or marker value or range of marker values from a patient having proteinuria, or that will develop proteinuria, or that has proteinuria that is manageable by known treatments, etc. Alternatively, the proteinuria reference element may be a negative reference/control, e.g. a sample or marker value or range of marker values from an individual that does not have proteinuria, or that is not at risk for developing proteinuria. Proteinuria reference elements are preferably the same type of sample or, if nonGly-suPAR analyte values, are obtained from the same type of sample as the sample that was employed to generate the nonGly-suPAR analyte value for the individual being monitored. For example, if the serum of an individual is being evaluated, the proteinuria reference element would preferably be of serum.

In certain embodiments, the obtained nonGly-suPAR analyte value is compared to a single proteinuria reference element to obtain information regarding the individual being tested for proteinuria. In other embodiments, the obtained nonGly-suPAR analyte value is compared to two or more proteinuria reference elements. For example, the obtained nonGly-suPAR analyte value may be compared to a negative reference and a positive reference to obtain confirmed information regarding if the individual will develop proteinuria. As another example, the obtained nonGly-suPAR analyte value may be compared to a reference that is representative of a proteinuria that is responsive to treatment and a reference that is representative of a proteinuria that is not responsive to treatment to obtain information as to whether or not the patient will be responsive to treatment.

The comparison of the obtained marker level representation to the one or more proteinuria reference elements may be performed using any convenient methodology, where a variety of methodologies are known to those of skill in the art. For example, those of skill in the art of ELISAs will know that ELISA data may be compared by, e.g. normalizing to standard curves, comparing normalized values, etc. The comparison step results in information regarding how similar or dissimilar the obtained marker level profile is to the control/reference profile(s), which similarity/dissimilarity information is employed to, for example, predict the onset of a proteinuria, diagnose proteinuria, monitor a proteinuria patient, etc. Methods of comparing marker level values are also described above. Similarity may be based on relative marker levels, absolute marker levels or a combination of both. In certain embodiments, a similarity determination is made using a computer having a program stored thereon that is designed to receive input for a marker level representation obtained from a subject, e.g., from a user, determine similarity to one or more reference elements, and return an proteinuria prognosis, e.g., to a user (e.g., lab technician, physician, pregnant individual, etc.). Further descriptions of computer-implemented aspects of the invention are described below. In certain embodiments, a similarity determination may be based on a visual comparison of the nonGly-suPAR analyte value to a range of proteinuria reference elements, e.g. a range of nonGly-suPAR analyte values, to determine the reference element that is most similar to that of the subject. Depending on the type and nature of the proteinuria reference element to which the obtained nonGly-suPAR analyte value is compared, the above comparison step yields a variety of different types of information regarding the cell/bodily fluid that is assayed. As such, the above comparison step can yield a positive/negative prediction of the onset of proteinuria, a positive/negative diagnosis of proteinuria, a characterization of a proteinuria, information on the responsiveness of a proteinuria to treatment, and the like.

In other embodiments, the nonGly-suPAR analyte value is employed directly, i.e., without comparison to a proteinuria reference element, to make a proteinuria assessment. For example, a patient may be predicted to develop proteinuria if the concentration of nonGly-suPAR in the patient's serum is greater than about 1ng/ml, e.g. 2ng/ml, 3 ng/ml, 4 ng/ml, 5 ng/ml, 7 ng/ml, 10ng/ml or greater.

In some embodiments, the subject methods of providing a proteinuria assessment, e.g. diagnosing proteinuria, prognosing proteinuria, monitoring the proteinuria, and the like, may comprise additional assessment(s) that are employed in conjunction with the subject nonGly-suPAR analyte value. For example, the subject methods may further comprise measuring one or more clinical parameters/factors associated with proteinuria, e.g. elevated levels of protein in urine, edema, and the like, wherein a positive outcome of the clinical assessment (i.e. the detection of one or more symptoms associated with proteinuria) is used in combination with the nonGly-suPAR analyte value to provide a proteinuria diagnosis, a proteinuria prognosis, to monitor the proteinuria, etc.

As another example, the subject methods of providing a proteinuria assessment may further comprise assessing one or more factors associated with the risk of developing proteinuria. Non-limiting examples of proteinuria risk factors include, for example, microalbuminuria, diabetes, hypertension, heritage (African American, American Indian, Hispanic American, Pacific Islander American), older age, weight, and a family history of kidney disease, wherein a positive outcome of the risk assessment (i.e. the determination of one or more risk factors associated with proteinuria) is used in combination with the nonGly-suPAR analyte value to provide a proteinuria diagnosis, a proteinuria prognosis, to monitor the proteinuria, etc.

The subject methods may be employed for a variety of different types of subjects. In many embodiments, the subjects are within the class mammalian, including the orders carnivore (e.g., dogs and cats), rodentia (e.g., mice, guinea pigs, and rats), lagomorpha (e.g. rabbits) and primates (e.g., humans, chimpanzees, and monkeys). In certain embodiments, the animals or hosts, i.e., subjects (also referred to herein as patients), are humans.

In some embodiments, the subject methods of providing a proteinuria assessment include providing a diagnosis, prognosis, or result of the monitoring. In some embodiments, the proteinuria assessment of the present disclosure is provided by providing, i.e. generating, a written report that includes the artisan's assessment, for example, the artisan's determination of whether the patient is currently affected by proteinuria, of the type, stage, or severity of the subject's proteinuria, etc. (a "proteinuria diagnosis"); the artisan's prediction of the patient's susceptibility to developing proteinuria, of the course of disease progression, of the patient's responsiveness to treatment, etc. (i.e., the artisan's "proteinuria prognosis"); or the results of the artisan's monitoring of the proteinuria. Thus, the subject methods may further include a step of generating or outputting a report providing the results of an artisan's assessment, which report can be provided in the form of an electronic medium (e.g., an electronic display on a computer monitor), or in the form of a tangible medium (e.g., a report printed on paper or other tangible medium). Any form of report may be provided, e.g. as known in the art or as described in greater detail below.

### REPORTS

A "report," as described herein, is an electronic or tangible document which includes report elements that provide information of interest relating to the assessment of a subject and its results. In some embodiments, a subject report includes at least a proteinuria marker representation, e.g. a proteinuria profile or a proteinuria score, as discussed in greater detail above. In some embodiments, a subject report includes at least an artisan's proteinuria assessment, e.g. proteinuria diagnosis, proteinuria prognosis, an analysis of a proteinuria monitoring, a treatment recommendation, etc. A subject report can be completely or partially electronically generated. A subject report can further include one or more of: 1) information regarding the testing facility; 2) service provider information; 3) patient data; 4) sample data; 5) an assessment report, which can include various information including: a) reference values employed, and b) test data, where test data can include, e.g., a suPAR level determination; 6) other features.

The report may include information about the testing facility, which information is relevant to the hospital, clinic, or laboratory in which sample gathering and/or data generation was conducted. Sample gathering can include obtaining a fluid sample, e.g. blood, urine, saliva, etc.; a tissue sample, e.g., a tissue biopsy, etc. from a subject. Data generation can include measuring the marker concentration in proteinuria patients versus healthy individuals, i.e. individuals that do not have and/or do not develop proteinuria. This information can include one or more details relating to, for example, the name and location of the testing facility, the identity of the lab technician who conducted the assay and/or who entered the input data, the date and time the assay was conducted and/or analyzed, the location where the sample and/or result data is stored, the lot number of the reagents (e.g., kit, etc.) used in the assay, and the like. Report fields with this information can generally be populated using information provided by the user.

The report may include information about the service provider, which may be located outside the healthcare facility at which the user is located, or within the healthcare facility. Examples of such information can include the name and location of the service provider, the name of the reviewer, and where necessary or desired the name of the individual who conducted sample gathering and/or data generation. Report fields with this information can generally be populated using data entered by the user, which can be selected from among pre-scripted selections (e.g., using a drop-down menu). Other service provider information in the report can include contact information for technical information about the result and/or about the interpretive report.

The report may include a patient data section, including patient medical history (which can include, e.g., age, race, serotype, prior preeclampsia episodes, and any other characteristics of the pregnancy), as well as administrative patient data such as information to identify the patient (e.g., name, patient date of birth (DOB), gender, mailing and/or residence address, medical record number (MRN), room and/or bed number in a healthcare facility), insurance information, and the like), the name of the patient's physician or other health professional who ordered the monitoring assessment and, if different from the ordering physician, the name of a staff physician who is responsible for the patient's care (e.g., primary care physician).

The report may include a sample data section, which may provide information about the biological sample analyzed in the monitoring assessment, such as the source of biological sample obtained from the patient (e.g. blood, urine, saliva, or type of tissue, etc.), how the sample was handled (e.g. storage temperature, preparatory protocols) and the date and time collected. Report fields with this information can generally be populated using data entered by the user, some of which may be provided as pre-scripted selections (e.g., using a drop-down menu). The report may include a results section. For example, the report may include a section reporting the results of a suPAR level determination assay (e.g., "1.5 nmol/liter nonGly-suPAR in serum"), or a calculated nonGly-suPAR analyte value.

The report may include an assessment report section, which may include information generated after processing of the data as described herein. The interpretive report can include a prediction of the likelihood that the subject will develop proteinuria. The interpretive report can include a diagnosis of proteinuria. The interpretive report can include a characterization of proteinuria. The assessment portion of the report can optionally also include a recommendation(s). For example, where the results indicate that proteinuria is likely, the recommendation can include a recommendation that diet be altered, blood pressure medicines administered, etc., as recommended in the art.

It will also be readily appreciated that the reports can include additional elements or modified elements. For example, where electronic, the report can contain hyperlinks which point to internal or external databases which provide more detailed information about selected elements of the report. For example, the patient data element of the report can include a hyperlink to an electronic patient record, or a site for accessing such a patient record, which patient record is maintained in a confidential database. This latter embodiment may be of interest in an in-hospital system or in-clinic setting. When in electronic format, the report is recorded on a suitable physical medium, such as a computer readable medium, e.g., in a computer memory, zip drive, CD, DVD, etc.

It will be readily appreciated that the report can include all or some of the elements above, with the proviso that the report generally includes at least the elements sufficient to provide the analysis requested by the user (e.g. a calculated nonGly-suPAR analyte value ; a prediction, diagnosis or characterization of proteinuria).

### REAGENTS, DEVICES, SYSTEMS AND KITS

Also provided are reagents, systems, devices and kits thereof for practicing one or more of the above-described methods. The subject reagents, systems and kits thereof may vary greatly. Reagents of interest include reagents specifically designed for use in producing the above-described nonGly-suPAR analyte values from a sample, for example, one or more detection elements, e.g. antibodies or peptides for the detection of protein, lectins for the detection of glycosylated protein , etc. Also encompassed are cocktails of reagents, e.g. compositions that comprise more than one detection elements, e.g. antibodies, peptide(s), lectin(s), etc. which may be used to detect the expression of one or more proteins, e.g. nonGly-suPAR and Gly-suPAR, nonGly-suPAR and total suPAR, Gly-suPAR and total suPAR, glycosylated protein and nonGly-suPAR, etc., simultaneously.

One type of reagent that is specifically tailored for generating a nonGly-suPAR analyte value is an antibody. For example, the antibody may bind specifically to nonglycosylated suPAR, e.g. in an ELISA format, in an xMAP™ microsphere format, on a proteomic array, in suspension for analysis by flow cytometry, by western blotting, by dot blotting, by immunohistochemistry, etc. As another example, the antibody may bind specifically to glycosylated suPAR. As a third example, the antibody may bind to both nonglycosylated and glycosylated suPAR, i.e. it binds total suPAR. Thus, for example, nonGly-suPAR levels may be evaluated by detecting nonGly-suPAR levels; or by detecting Gly-suPAR levels and total suPAR levels, and evaluating nonGly-suPAR levels by calculating the difference between Gly-suPAR and total suPAR levels; and so on. Other reagents that may be used in conjunction with such antibodies include lectins, e.g. as described above. Methods for using antibodies and lectins are well understood in the art, and are described in greater detail above. Such affinity reagents may be provided in solution. Alternatively, they may be provided pre-bound to a solid matrix, for example, the well of a multi-well dish, the surface of an xMAP microsphere, a nitrocellulose support, and the like.

In some instances, a device, i.e., a proteinuria assessment device, is provided. As used herein, the term "device" refers to a piece of equipment that may be employed in the evaluation of nonGly-suPAR levels in a sample, for example, a dipstick, a plate, or an array that comprises one or more detection elements, e.g. one or more antibodies, one or more peptides, one or more lectins, etc. which may be used to measure nonGly-suPAR levels. In some embodiments, the device comprises one or more nonGly-suPAR detection reagents. In some embodiments, the device is configured to obtain a nonGly-suPAR analyte value for a sample from a subject, and output a proteinuria assessment for the subject based on the nonGly-suPAR analyte value.

In some instances, a system is provided. As used herein, the term "system" refers to a collection of reagents and/or devices, however compiled, e.g., by purchasing the collection of reagents from the same or different sources.

In some instances, a kit is provided. As used herein, the term "kit" refers to a collection of reagents and/or devices provided, e.g., sold, together. For example, the antibody-based detection platform for detecting nonGly-suPAR levels may be coupled with histochemical reagents. As another example, histochemical reagents may be provided with a proteinuria assessment device, e.g. as described below. As a third example, a proteinuria assessment device may be provided with references or reference reports. It is envisioned that such combinations of reagents and devices may be provided as kits for personalized proteinuria care.

The systems and kits of the subject invention may include the above-described suPAR-specific antibody collections, glycosylated protein-specific lectins, and/or proteinuria assessment devices. The systems and kits may further include one or more additional reagents employed in the various methods, such various buffer mediums, e.g. hybridization and washing buffers, prefabricated probe arrays, labeled probe purification reagents and components, like spin columns, etc., signal generation and detection reagents, e.g. labeled secondary antibodies, streptavidin-alkaline phosphatase conjugate, chemifluorescent or chemiluminescent substrate, and the like.

The subject systems and kits may also include one or more proteinuria references, which element is, in many embodiments, a reference or control sample or marker value that can be employed, e.g., by a suitable experimental or computing means, to make a proteinuria assessment based on an "input" marker value, e.g., that has been determined with the above described detection element. Representative proteinuria references include samples from an individual known to have or not have proteinuria, databases of nonGly-suPAR analyte values, e.g., reference or control profiles or scores, and the like, as described above.

In addition to the above components, the subject kits will further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed site. Any convenient means may be present in the kits.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

General methods in molecular and cellular biochemistry can be found in such standard textbooks as Molecular Cloning: A Laboratory Manual, 3rd Ed. (Sambrook et al., HaRBor Laboratory Press 2001); Short Protocols in Molecular Biology, 4th Ed. (Ausubel et al. eds., John Wiley & Sons 1999); Protein Methods (Bollag et al., John Wiley & Sons 1996); Nonviral Vectors for Gene Therapy (Wagner et al. eds., Academic Press 1999); Viral Vectors (Kaplift & Loewy eds., Academic Press 1995); Immunology Methods Manual (I. Lefkovits ed., Academic Press 1997); and Cell and Tissue Culture: Laboratory Procedures in Biotechnology (Doyle & Griffiths, John Wiley & Sons 1998). Reagents, cloning vectors, and kits for genetic manipulation referred to in this disclosure are available from commercial vendors such as BioRad, Stratagene, Invitrogen, Sigma-Aldrich, and ClonTech.

### EXAMPLE 1

### suPAR levels in plasma do not correlate with β₃ integrin activation

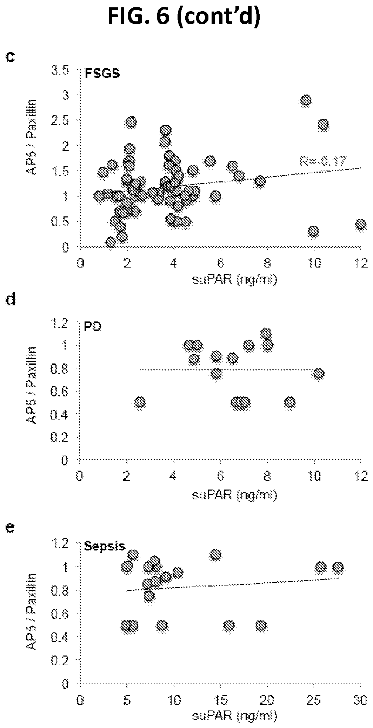

To investigate the role of suPAR in FSGS, suPAR levels were measured in FSGS patients during a standard check-up visit. As controls, serums from patients on peritoneal dialysis (PD), and those with sepsis, were used since those three pathological conditions have been associated with increased suPAR levels. As seen before, serums of patients with FSGS exhibited increased suPAR levels, whereas the suPAR levels were even more pronounced in patients on peritoneal dialysis, or those with sepsis (FIG. 6, Panel A). Those data are consistent with findings that loss of kidney function leads to increased plasma suPAR levels, as well as role of suPAR during infection. Based on the suPAR levels, FSGS patients could be separated in two distinct subgroups: those with levels above 3 ng/ml (mean = 5.17±1.8; 73%), and those with levels lower than 3 ng/ml (mean = 1.84±0.35, 27%). In fact, FSGS patients with levels lower than 3 ng/ml exhibited suPAR levels similar to healthy individuals (mean = 1.82±0.32).

It has been suggested that the pathological effect of suPAR on podocytes is via activation on standardly low levels of β₃ integrin signaling. Therefore, we next tested whether suPAR levels in serums of FSGS patients correlated with β₃ integrin activation. In order to compare β₃ integrin activation induced by different serums, the original assay was modified so that it measures the ratio of levels of activated integrin (AP5 staining) to levels of the total amount of focal adhesions (FAs, determined by Paxillin staining) (FIG. 6, Panel B). Therefore, alterations in number of FAs due to alterations in cell size or general diversity between cells in culture are removed from the analysis. The β₃ activation assay was done in a blind manner without any prior knowledge of any pathological condition of the patients.

There was no significant difference in the amount of activating integrin by four different healthy serums (HS) when compared to addition of 10% fetal bovine serum (FBS) standardly used to grow podocytes in culture, demonstrating that addition of healthy serum does not result in potent β₃ integrin activation. Thus, the amounts of active β₃ integrin in the presence of FSGS serums were compared to that of healthy serum, which was used as a negative control. While a number of serums clearly induced potent β₃ integrin activation, no statistically significant correlation was found between suPAR levels and levels of integrin activation (FIG. 6, Panel C). In addition, analysis of FSGS serums showed that serum addition did not alter levels of mRNA for αvβ3 or αvβ1 integrin, suggesting that observed β3 activation by immunofluorescence was due to changes in conformational state of integrin and not due to alteration in overall levels of β3 integrin in the cell culture. Furthermore, suPAR levels or AP5 activation could not separate primary from non-recurrent and recurrent FSGS. Surprisingly, β3 integrin activation was not was induced by the serums of patients on PD or sepsis (FIG. 6, Panels D and E), though those serums had the highest suPAR levels. Together, these data demonstrate that although a subset of FSGS serums did potently activate β3 integrin, suPAR levels in the plasma could not predict that activation.

### EXAMPLE 2

Focal segmental glomerulosclerosis (FSGS) is a glomerular disease characterized by marked proteinuria, often coupled with steroid resistance, hypertension, and high incidence of progression to renal failure. It is the most common progressive glomerular disease in children and it accounts for 20-25% of idiopathic nephrotic syndrome in adults. The etiology of FSGS is defined by its histological pattern while its mechanism(s) is still unclear. Renal biopsy is the only means to make the clinical diagnosis.

Histological and human monogenetic data strongly implicate podocyte dysfunction as a root cause in FSGS. Podocytes are terminally differentiated cells of the kidney glomerulus that are essential for function of the glomerular filter. Their function is primarily based on their intricate structure, in particular their foot processes (FPs), which are actin driven membrane extensions. Loss of these membrane extensions (referred to as FP effacement) is tightly connected to the presence of albumin in the urine (proteinuria), a first step injury that often leads to loss of podocytes and development of chronic kidney disease and end stage renal failure.

Current therapy for FSGS results in full or partial remission in only approximately 50% of patients. Treatments that are used include inhibitors of the immune response such as corticosteroids with or without cyclophosphamide, cyclosporine, mycophenolate mofetil (MMF), and rituximab. If proteinuria can be reduced by these agents or by non-specific therapies such as angiotensin converting enzyme inhibitors (ACEIs), angiotensin receptor blockers (ARBs), and/or lipid lowering agents, progression of renal dysfunction is slowed. When all these treatments fail and the patient's kidney function declines, the final available treatments are hemodialysis and/or kidney transplantation. Unfortunately, in approximately 30% of adults and up to 80% of pediatric FSGS patients that receive kidney transplants, the disease will recur, causing graft failure. A clear understanding of the molecular mechanisms that drive recurrent FSGS are essential for development of novel treatments, which should allow patients to keep their original kidneys and prevent the necessity for kidney transplants.

### Immunodepletion of suPAR from the human plasma decreases the amount of activated αᵥβ₃ integrin in culture.

It has been shown that human plasma from FSGS patients comprises elevated levels of suPAR which induces the activation of αᵥβ₃ integrin in cultured human podocytes (Wei, C., et al., "Circulating urokinase receptor as a cause of focal segmental glomerulosclerosis", Nature Medicine (2011), 17:952-960). For example, as demonstrated in Figure 1, addition of 10% human serum from patients with FSGS (-10 ng/ml of suPAR) to cultured human podocytes resulted in potent αᵥβ₃ integrin activation (Fig. 1, "FSGS serum" panels), as evidenced by immunofluorescence with an antibody that detects the active conformation of β₃ integrin (AP5, GTI Diagnostics). Addition of 10% fetal bovine serum (Fig. 1, "FBS" panels) in serum-free media (SFM) cultured human podocytes did not result in positive signal, indicating that activation was dependent on the presence of human serum. To demonstrate that the observed activation was due to the presence of suPAR in the human plasma, suPAR was quantitatively removed using mouse monoclonal anti-suPAR antibody ATN615 provided by Tactic Pharma (Highland Park, IL). The removal of suPAR from plasma was monitored using ELISA from R&D (Minneapolis, MN), which showed that ATN615 Ab removed >99% of suPAR from human plasma. Importantly, quantitative depletion of suPAR abolished the ability of FSGS plasma to activate αᵥβ₃ integrin (Fig. 1A and Fig. 1B for quantification).

Interestingly, mock depletion of human FSGS plasma by incubation of human plasma with only protein G-agarose beads also reduced integrin activation (Fig. 1B). ELISA assays illustrate that this procedure results in lowering the level of suPAR in plasma approximately 20-50% (mock sample standardly contained -3.5 ng/ml of suPAR, n=3). Thus, the impairment of mock-depleted human plasma to induce potent integrin activation could be explained by the lowered amount of suPAR in those samples. These data suggest that suPAR might bind protein G and protein A-agarose.

### suPAR binds protein A and protein G-agarose.

In order to directly test the ability of suPAR to bind protein A- and protein G-agarose, suPAR (R&D, Minneapolis, MN) was incubated with protein G-agarose beads or with protein A-agarose beads for 8 hours (Fig. 1C, Initial). Levels of suPAR that were loaded onto the column as well as those that flowed through the column were detected using Western blot analysis and R&D ELISA assay (of note, concentrations of the proteins in Fig. 1C do not correspond to levels of proteins loaded onto the gel, but those used in the experiment). suPAR that was bound to the column was detected by Western blot. Together, these data show that suPAR is capable of binding both protein A- and protein G-agarose beads directly. These data are consistent with suPAR acting as a pathogenic factor in recurrent FSGS which can be partially removed by immunoadsorption.

### Expression of non-glycosylated suPAR in E. coli.

In order to test our hypothesis that FSGS is driven by a specific form of suPAR, which could either be a specific fragment or a modified glycosylation pattern or both, we have expressed different suPAR fragments in E. coli (Fig. 2A). Proteins expressed in bacteria will not be glycosylated, thus allowing us to test effects of non-glycosylated suPAR (nonGly-suPAR) fragments for their ability to activate αvβ3 integrin in cultured human podocytes. As shown in Fig. 2C, all fragments were efficiently expressed in E. coli (Coomassie staining of total bacterial proteins). While the majority of the proteins were highly insoluble, most likely due to expression in bacteria, we were able to purify sufficient amounts of soluble fragments and full-length suPAR. Although the proteins were expressed in BL21 DE3 strain of E. coli, which is modified so that it does not contain high levels of bacterial endotoxins, all recombinant proteins were further purified by incubation with High-Capacity Endotoxin Removal Resin (Pierce, Rackford, IL).

### NonGly-suPAR is a potent activator of αᵥβ₃ integrin in culture.

It has also been shown that addition of recombinant suPAR increases αvβ3 activation in cultured human podocytes (Wei, C., et al., *supra*). However, the levels needed to observe activation were 200 times higher (1 µg/ml) than those observed in human plasma (5-10 ng/ml), thus being physiologically questionable. The recombinant suPAR (R&D) used in these experiments was a recombinant mouse chimeric form of suPAR that comprising an Fc fragment (m-suPAR-Fc). Thus, it was theoretically possible that such modified mouse suPAR was not an efficient activator of αᵥβ₃ integrin in human podocytes. We repeated these original experiments using physiologically comparable levels of glycosylated C-terminally His-tagged human suPAR DI-DIII (Gly-suPAR) from R&D (Accession #Q03405) expressed in mouse myeloma cell line. As shown in Fig. 3, human podocytes were serum starved in serum free media for 24 h. 50 ng/ml of human recombinant Gly-suPAR was then added for overnight treatment. Activated αᵥβ₃ integrin was detected using AP5 antibody and focal adhesions were visualized using an anti-paxillin Ab. While we noticed the appearance of a large number of focal complexes that co-localized with activated αᵥβ₃ integrin (data not shown), addition of physiological levels of Gly-suPAR did not result in potent activation of αᵥβ₃ integrin (Fig. 3B for quantification). These data demonstrate that physiological levels of Gly-suPAR (between 4-15 ng/ml) are not sufficient to induce the amount of activation observed by addition of FSGS plasma (compare Fig. 3 with Fig. 1). In agreement with in vitro data, injection of mouse suPAR-Fc in wild-type mice did not lead to significant proteinuria (Wei, C., et al., *supra*)*.* Together, these data strongly suggest that FSGS is not driven by high levels of wild type full length Gly-suPAR.

Strikingly, addition of physiologic levels of nonglycosylated human suPAR (NonGly-suPAR, 5 ng/ml) resulted in potent activation of αᵥβ₃ integrin (Fig 3a, bottom panels). The extent of activation was similar to that observed using FSGS plasma (Fig. 1). These data strongly suggest that loss of glycosylation renders suPAR a more potent activator of αᵥβ₃ integrin, e.g. by increasing the affinity of suPAR for αvβ3 integrin.

### Administration of NonGly-suPAR to wild-type mice induces proteinuria.

The data above demonstrate that NonGly-suPAR is more potent activator of αᵥβ₃ integrin than Gly-suPAR. They also demonstrate that observed αᵥβ₃ integrin activation in human podocytes by FSGS plasma is due to the presence of pathological form of suPAR. It is believed that αᵥβ₃ integrin activation corresponds to FP effacement and proteinuria (Wei, C., et al., "Modification of kidney barrier function by the urokinase receptor", Nature medicine (2003), 14:55-63; Wei, C., et al., "Circulating urokinase receptor as a cause of focal segmental glomerulosclerosis", Nature Medicine (2011), 17:952-960). To further test this hypothesis, NonGly-suPAR was injected into the tail vein of BALB/C mice, and levels of albumin and creatinine were determined using ELISA assays. As shown in Fig. 4, administration of as little as 80 ng of NonGly-suPAR per mouse induced a statistically significant increase in proteinuria. In contrast, administration of 20 µg of Gly-suPAR (R&D) had no effect. Proteinuria was reversible by stopping further suPAR injection but appears to persist after prolonged injections (>1 week). Administration of 20 µg of Gly-suPAR (R&D) had no effect. An average mouse has ∼3 ml of blood which is ∼ 1.5 ml of plasma. Based on human data, mice may have ∼5 ng/ml of suPAR. Thus, a 5-10 fold increase in suPAR (25-50 ng/ml) is expected to induce proteinuria, based on human data. At a concentration of 80 ng per mouse (53 ng/ml plasma), these experiments are in the range of suPAR concentration that is expected to induce proteinuria. While proteinuria was not dramatic, and not in the nephrotic range, the observed ∼3 fold increase is consistent with the increase in proteinuria in Plaur-/- mice (Wei C., et al., "Circulating urokinase receptor as a cause of focal segmental glomerulosclerosis", Nature medicine (2011), 17:952-960), as well as level of proteinuria in rats injected with human samples (Sharm, M and Slavin VJ 1999, JASN 10). Thus, loss of glycosylation renders suPAR a more potent activator of αᵥβ₃ integrin in podocytes, which drives FP effacement and proteinuria.

### ELISA assay used to measure suPAR levels in patients recognizes only Gly-suPAR.

One of the major obstacles in the identification of the specific form of suPAR in FSGS patients is the closely held knowledge of the antigenic sites recognized by commercially available ELISA assays. This information is generally considered proprietary and thus unavailable to researchers. We used our E. coli expressed suPAR fragments in addition to fully glycosylated human suPAR (R&D) to determine specific domains that are recognized by antibodies in a particular ELISA assay (R&D). Mouse monoclonal anti-suPAR antibody (R&D) that is also commonly used to measure suPAR levels in human recognized primarily glycosylated suPAR (Fig. 5). Thus, the antigenic site recognized by R&D antibody is a combination of amino acids and glycosylation, and the R&D antibody may be said to recognize glycosylated suPAR.

## Claims

1. A method for providing a proteinuria assessment for a subject, the method comprising:
obtaining a non-glycosylated soluble urokinase plasminogen-type activator receptor (nonGly-suPAR) analyte value from a biological sample of a subject comprising the use of a proteinuria assessment device configured to obtain a nonGly-suPAR analyte value for a sample from a subject and output a proteinuria assessment for the subject based on the nonGly-suPAR analyte value, the device comprising one or more nonGly-suPAR detection reagents,
wherein the nonGly-suPAR analyte is a nonGly-suPAR variant that contains three N-linked glycans, two N-linked glycans, one N-linked glycan, or no N-linked glycan;
providing a proteinuria assessment based on the obtained nonGly-suPAR analyte value comprising comparing the nonGly-suPAR analyte value to a reference,
wherein the reference is representative of an individual that does not have proteinuria, wherein an elevated nonGly-suPAR analyte value in the sample relative to the reference indicates that the subject has developed proteinuria or is at risk for developing proteinuria; and
providing the proteinuria assessment based on the comparison.

2. The method according to claim 1, wherein the assessment is a proteinuria prognosis, a proteinuria diagnosis, or an assessment of the responsiveness of an individual to proteinuria treatment.

3. The method of claims 1 or 2, wherein the proteinuria assessment is proteinuria diagnosis and wherein the patient is diagnosed as having proteinuria when the level of nonGly-suPAR is at least 1.5 times greater than a control level of nonGly-suPAR in a sample not associated with the proteinuria phenotype; and
optionally wherein obtaining a nonGly-suPAR value is performed using an antibody specific for the nonGly-suPAR analyte .

4. The method according to any one of claims 1 to 3, wherein the subject has normal glomerular filtration rate (GFR) and normoalbuminuria; or wherein the individual has microalbuminuria or a kidney disease, wherein optionally the kidney disease is FSGS or diabetic kidney disease.

5. The method according to any one of claims 1 to 4, wherein obtaining a nonGly-suPAR analyte value comprises measuring the level of a nonGly-suPAR analyte selected from the group consisting of: nonGly-suPAR(I-III), nonGly-suPAR(II-III), nonGly- suPAR(I), and any combination thereof.

6. The method according to any one of claims 1 to 5, wherein the sample from the subject is a blood sample or a urine sample.

7. A proteinuria assessment device configured to
obtain a non-glycosylated soluble urokinase plasminogen-type activator receptor (nonGly-suPAR) analyte value from a biological sample of a subject and output a proteinuria assessment for the subject based on the nonGly-suPAR analyte value, the device comprising one or more nonGly-suPAR detection reagents,
wherein the nonGly-suPAR analyte is a nonGly-suPAR variant that contains three N-linked glycans, two N-linked glycans, one N-linked glycan, or no N-linked glycan; and
providing a proteinuria assessment based on the obtained nonGly-suPAR analyte value comprising comparing the nonGly-suPAR analyte value to a reference,
wherein the reference is representative of an individual that does not have proteinuria, wherein an elevated nonGly-suPAR analyte value in the sample relative to the reference indicates that the subject has developed proteinuria or is at risk for developing proteinuria; and
providing the proteinuria assessment based on the comparison.

8. The proteinuria assessment device according to claim 7, wherein the one or more nonGly-suPAR analyte detection reagents is bound to a solid support, or
wherein the one or more nonGly-suPAR analyte detection reagents comprises a detection element specific for glycans and a detection element specific for suPAR analyte, or
wherein the one or more nonGly-suPAR analyte detection reagents comprises a detection element specific for Gly-suPAR analyte, and a detection element specific for suPAR analyte.

## Patentansprüche

1. Verfahren zur Bereitstellung eines Proteinurietestergebnisses für ein Subjekt, wobei das Verfahren Folgendes umfasst:
die Gewinnung eines Analytenwertes für den Rezeptor des nichtglykosylierten löslichen Urokinase-Plasminogenaktivators (Non-Glycosylated Soluble Urokinase Plasminogen-Type Activator Receptor, nonGly-suPAR) von einer biologischen Probe eines Subjekts, umfassend die Verwendung eines für die Gewinnung eines nonGly-suPAR-Analytenwertes für eine Probe von einem Subjekt und die Ausgabe eines Proteinurietestergebnisses für das Subjekt basierend auf dem nonGly-suPAR-Analytenwert ausgelegten Proteinurietestgeräts, wobei das Gerät ein oder mehrere Reagenzien zum Nachweis von nonGly-suPAR umfasst,
wobei es sich bei dem nonGly-suPAR-Analyten um eine nonGly-suPAR-Variante handelt, die drei N-gebundene Glykane, zwei N-gebundene Glykane, einen N-gebundenen Glykan oder keinen N-gebundenen Glykan enthält,
die Bereitstellung eines Proteinurietestergebnisses basierend auf dem erhaltenen nonGly-suPAR-Analytenwert, umfassend den Vergleich des nonGly-suPAR-Analytenwertes mit einem Vergleichswert,
wobei der Vergleichswert repräsentativ für ein Individuum ist, das nicht an Proteinurie leidet, wobei ein erhöhter nonGly-suPAR-Analytenwert in der Probe, bezogen auf den Vergleichswert, darauf hinweist, dass bei dem Subjekt Proteinurie aufgetreten ist oder dass bei dem Subjekt das Risiko des Auftretens von Proteinurie besteht, und
die Bereitstellung des Proteinurietestergebnisses basierend auf dem Vergleich.

2. Verfahren nach Anspruch 1, wobei das Testergebnis eine Proteinurieprognose, eine Proteinuriediagnose oder ein Testergebnis für das Ansprechen eines Individuums auf eine Proteinuriebehandlung ist.

3. Verfahren nach Anspruch 1 oder 2, wobei es sich bei dem Proteinurietestergebnis um eine Proteinuriediagnose handelt und wobei der Patient als an Proteinurie leidend diagnostiziert wird, wenn die Konzentration an nonGly-suPAR mindestens 1,5-fach größer ist als eine Vergleichskonzentration an nonGly-suPAR in einer nicht mit dem Proteinurie-Phänotyp assoziierten Probe, und
wobei die Gewinnung eines nonGly-suPAR-Wertes gegebenenfalls mit einem für den nonGly-suPAR-Analyten spezifischen Antikörper erfolgt.

4. Verfahren nach einem der Ansprüche 1 bis 3, wobei das Subjekt eine normale glomeruläre Filtrationsrate (GFR) und Normoalbuminurie hat oder wobei das Individuum Mikroalbuminurie oder eine Nierenkrankheit hat, wobei es sich bei der Nierenkrankheit gegebenenfalls um FSGS oder diabetische Nierenkrankheit handelt.

5. Verfahren nach einem der Ansprüche 1 bis 4, wobei die Gewinnung eines nonGly-suPAR-Analytenwertes das Messen der Konzentration eines nonGly-suPAR-Analyten ausgewählt aus der Gruppe bestehend aus nonGly-suPAR(I-III), nonGly-suPAR(II-III), nonGly-suPAR(I) und einer beliebigen Kombination davon umfasst.

6. Verfahren nach einem der Ansprüche 1 bis 5, wobei es sich bei der Probe von dem Subjekt um eine Blutprobe oder eine Urinprobe handelt.

7. Proteinurietestgerät, ausgelegt für
die Gewinnung eines Analytenwertes für den Rezeptor des nichtglykosylierten löslichen Urokinase-Plasminogenaktivators (nonGly-suPAR) von einer biologischen Probe eines Subjekts und Ausgabe eines Proteinurietestergebnisses für das Subjekt basierend auf dem nonGly-suPAR-Analytenwert, wobei das Gerät ein oder mehrere Reagenzien zum Nachweis von nonGly-suPAR umfasst,
wobei es sich bei dem nonGly-suPAR-Analyten um eine nonGly-suPAR-Variante handelt, die drei N-gebundene Glykane, zwei N-gebundene Glykane, einen N-gebundenen Glykan oder keinen N-gebundenen Glykan enthält, und
die Bereitstellung eines Proteinurietestergebnisses basierend auf dem erhaltenen nonGly-suPAR-Analytenwert, umfassend den Vergleich des nonGly-suPAR-Analytenwertes mit einem Vergleichswert,
wobei der Vergleichswert repräsentativ für ein Individuum ist, das nicht an Proteinurie leidet, wobei ein erhöhter nonGly-suPAR-Analytenwert in der Probe, bezogen auf den Vergleichswert, darauf hinweist, dass bei dem Subjekt Proteinurie aufgetreten ist oder dass bei dem Subjekt das Risiko des Auftretens von Proteinurie besteht, und
die Bereitstellung des Proteinurietestergebnisses basierend auf dem Vergleich.

8. Proteinurietestgerät nach Anspruch 7, wobei das eine oder die mehreren nonGly-suPAR-Analytendetektionsreagenzien an einen festen Träger gebunden ist/sind oder
wobei das eine oder die mehreren nonGly-suPAR-Analytendetektionsreagenzien ein für Glykane spezifisches Detektionselement und ein für suPAR-Analyten spezifisches Detektionselement umfasst/umfassen oder
wobei das eine oder die mehreren nonGly-suPAR-Analytendetektionsreagenzien ein für Gly-suPAR-Analyten spezifisches Detektionselement und ein für suPAR-Analyten spezifisches Detektionselement umfasst/umfassen.

## Revendications

1. Procédé de fourniture d'une évaluation de la protéinurie pour un sujet, le procédé comprenant :
l'obtention d'une valeur d'analyte de récepteur d'activateur du plasminogène de type urokinase soluble, non glycosylé (nonGly-suPAR) à partir d'un échantillon biologique d'un sujet comprenant l'utilisation d'un dispositif d'évaluation de protéinurie configuré pour obtenir une valeur d'analyte nonGly-suPAR pour un échantillon d'un sujet et délivrer en sortie une évaluation de la protéinurie pour le sujet sur la base de la valeur d'analyte nonGly-suPAR, le dispositif comprenant un ou plusieurs réactifs de détection de nonGly-suPAR,
dans lequel l'analyte nonGly-suPAR est un variant de nonGly-suPAR qui contient trois N-glycanes, deux N-glycanes, un N-glycane ou aucun N-glycane ;
la fourniture d'une évaluation de la protéinurie sur la base de la valeur d'analyte nonGly-suPAR obtenue comprenant la comparaison de la valeur d'analyte nonGly-suPAR à une référence,
où la référence est représentative d'un individu qui ne présente pas une protéinurie, dans lequel une valeur d'analyte nonGly-suPAR élevée dans l'échantillon par rapport à la référence indique que le sujet a développé une protéinurie ou est à risque de développer une protéinurie ; et
la fourniture de l'évaluation de la protéinurie sur la base de la comparaison.

2. Procédé selon la revendication 1, dans lequel l'évaluation est un pronostic de protéinurie, un diagnostic de protéinurie ou une évaluation de la réponse d'un individu au traitement de la protéinurie.

3. Procédé selon les revendications 1 ou 2, dans lequel l'évaluation de la protéinurie est un diagnostic de protéinurie et dans lequel le patient est diagnostiqué comme ayant une protéinurie lorsque le taux de nonGly-suPAR est au moins 1,5 fois plus élevé qu'un taux témoin de nonGly-suPAR dans un échantillon non associé au phénotype de protéinurie ; et
facultativement dans lequel l'obtention d'une valeur nonGly-suPAR est effectuée en utilisant un anticorps spécifique pour l'analyte nonGly-suPAR.

4. Procédé selon l'une quelconque des revendications 1 à 3, dans lequel le sujet présente un débit de filtration glomérulaire (DFG) et une normoalbuminurie ; ou dans lequel l'individu présente une microalbuminurie ou une maladie rénale, dans lequel, facultativement, la maladie rénale est HSF ou une maladie rénale diabétique.

5. Procédé selon l'une quelconque des revendications 1 à 4, dans lequel l'obtention d'une valeur d'analyte nonGly-suPAR comprend la mesure du taux d'un analyte nonGly-suPAR choisi dans le groupe constitué de : nonGly-suPAR(I-III), nonGly-suPAR(II-III), nonGly-suPAR(I), et une combinaison quelconque de ceux-ci.

6. Procédé selon l'une quelconque des revendications 1 à 5, dans lequel l'échantillon du sujet est un échantillon de sang ou un échantillon d'urine.

7. Dispositif d'évaluation de la protéinurie configuré pour obtenir une valeur d'analyte de récepteur d'activateur du plasminogène de type urokinase soluble, non glycosylé (nonGly-suPAR) à partir d'un échantillon biologique d'un sujet et délivrer en sortie une évaluation de la protéinurie pour le sujet sur la base de la valeur d'analyte nonGly-suPAR, le dispositif comprenant un ou plusieurs réactifs de détection de nonGly-suPAR,
dans lequel l'analyte nonGly-suPAR est un variant de nonGly-suPAR qui contient trois N-glycanes, deux N-glycanes, un N-glycane ou aucun N-glycane ; et
la fourniture d'une évaluation de la protéinurie sur la base de la valeur d'analyte nonGly-suPAR obtenue comprenant la comparaison de la valeur d'analyte nonGly-suPAR à une référence,
où la référence est représentative d'un individu qui ne présente pas une protéinurie, dans lequel une valeur d'analyte nonGly-suPAR élevée dans l'échantillon par rapport à la référence indique que le sujet a développé une protéinurie ou est à risque de développer une protéinurie ; et
la fourniture de l'évaluation de la protéinurie sur la base de la comparaison.

8. Dispositif d'évaluation de la protéinurie selon la revendication 7, dans lequel les un ou plusieurs réactifs de détection d'analyte nonGly-suPAR sont liés un support solide, ou
dans lequel les un ou plusieurs réactifs de détection d'analyte nonGly-suPAR comprennent un élément de détection spécifique pour les glycanes et un élément de détection spécifique pour l'analyte suPAR, ou
dans lequel les un ou plusieurs réactifs de détection d'analyte nonGly-suPAR comprennent un élément de détection spécifique pour l'analyte Gly-suPAR, et un élément de détection spécifique pour l'analyte suPAR.


### SEQUENCE LISTING

<110> The General Hospital Corporation Rush University Medical Center Sever, Sanja Reiser, Jochen
<120> NON-GLYCOSYLATED suPAR BIOMARKERS AND USES THEREOF
<130> MGHS-001WO
<150> US 61/861,226
   <151> 2013-08-01
<160> 2
<170> PatentIn version 3.5
<210> 1
   <211> 1570
   <212> DNA
   <213> Homo sapiens
<400> 1
   gccgagccag ccccttcacc accagccggc cgcgccccgg gaagggaagt ttgtggcgga      60
   ggaggttcgt acgggaggag ggggaggcgc ccacgcatct ggggctgact cgctctttcg     120
   caaaacgtct gggaggagtc cctggggcca caaaactgcc tccttcctga ggccagaagg     180
   agagaagacg tgcagggacc ccgcgcacag gagctgccct cgcgacatgg gtcacccgcc     240
   gctgctgccg ctgctgctgc tgctccacac ctgcgtccca gcctcttggg gcctgcggtg     300
   catgcagtgt aagaccaacg gggattgccg tgtggaagag tgcgccctgg gacaggacct     360
   ctgcaggacc acgatcgtgc gcttgtggga agaaggagaa gagctggagc tggtggagaa     420
   aagctgtacc cactcagaga agaccaacag gaccctgagc tatcggactg gcttgaagat     480
   caccagcctt accgaggttg tgtgtgggtt agacttgtgc aaccagggca actctggccg     540
   ggctgtcacc tattcccgaa gccgttacct cgaatgcatt tcctgtggct catcagacat     600
   gagctgtgag aggggccggc accagagcct gcagtgccgc agccctgaag aacagtgcct     660
   ggatgtggtg acccactgga tccaggaagg tgaagaaggg cgtccaaagg atgaccgcca     720
   cctccgtggc tgtggctacc ttcccggctg cccgggctcc aatggtttcc acaacaacga     780
   caccttccac ttcctgaaat gctgcaacac caccaaatgc aacgagggcc caatcctgga     840
   gcttgaaaat ctgccgcaga atggccgcca gtgttacagc tgcaagggga acagcaccca     900
   tggatgctcc tctgaagaga ctttcctcat tgactgccga ggccccatga atcaatgtct     960
   ggtagccacc ggcactcacg aaccgaaaaa ccaaagctat atggtaagag gctgtgcaac    1020
   cgcctcaatg tgccaacatg cccacctggg tgacgccttc agcatgaacc acattgatgt    1080
   ctcctgctgt actaaaagtg gctgtaacca cccagacctg gatgtccagt accgcagtgg    1140
   ggctgctcct cagcctggcc ctgcccatct cagcctcacc atcaccctgc taatgactgc    1200
   cagactgtgg ggaggcactc tcctctggac ctaaacctga aatccccctc tctgccctgg    1260
   ctggatccgg gggacccctt tgcccttccc tcggctccca gccctacaga cttgctgtgt    1320
   gacctcaggc cagtgtgccg acctctctgg gcctcagttt tcccagctat gaaaacagct    1380
   atctcacaaa gttgtgtgaa gcagaagaga aaagctggag gaaggccgtg ggccaatggg    1440
   agagctcttg ttattattaa tattgttgcc gctgttgtgt tgttgttatt aattaatatt    1500
   catattattt attttatact tacataaaga ttttgtacca gtggacaagg ccaaaaaaaa    1560
   aaaaaaaaaa                                                           1570
<210> 2
   <211> 335
   <212> PRT
   <213> Homo sapiens
<400> 2
   Met Gly His Pro Pro Leu Leu Pro Leu Leu Leu Leu Leu His Thr Cys
   1               5                   10                  15
   Val Pro Ala Ser Trp Gly Leu Arg Cys Met Gln Cys Lys Thr Asn Gly
               20                  25                  30
   Asp Cys Arg Val Glu Glu Cys Ala Leu Gly Gln Asp Leu Cys Arg Thr
           35                  40                  45
   Thr Ile Val Arg Leu Trp Glu Glu Gly Glu Glu Leu Glu Leu Val Glu
       50                  55                  60
   Lys Ser Cys Thr His Ser Glu Lys Thr Asn Arg Thr Leu Ser Tyr Arg
   65                  70                  75                  80
   Thr Gly Leu Lys Ile Thr Ser Leu Thr Glu Val Val Cys Gly Leu Asp
                   85                  90                  95
   Leu Cys Asn Gln Gly Asn Ser Gly Arg Ala Val Thr Tyr Ser Arg Ser
               100                 105                 110
   Arg Tyr Leu Glu Cys Ile Ser Cys Gly Ser Ser Asp Met Ser Cys Glu
           115                 120                 125
   Arg Gly Arg His Gln Ser Leu Gln Cys Arg Ser Pro Glu Glu Gln Cys
       130                 135                 140
   Leu Asp Val Val Thr His Trp Ile Gln Glu Gly Glu Glu Gly Arg Pro
   145                 150                 155                 160
   Lys Asp Asp Arg His Leu Arg Gly Cys Gly Tyr Leu Pro Gly Cys Pro
                   165                 170                 175
   Gly Ser Asn Gly Phe His Asn Asn Asp Thr Phe His Phe Leu Lys Cys
               180                 185                 190
   Cys Asn Thr Thr Lys Cys Asn Glu Gly Pro Ile Leu Glu Leu Glu Asn
           195                 200                 205
   Leu Pro Gln Asn Gly Arg Gln Cys Tyr Ser Cys Lys Gly Asn Ser Thr
       210                 215                 220
   His Gly Cys Ser Ser Glu Glu Thr Phe Leu Ile Asp Cys Arg Gly Pro
   225                 230                 235                 240
   Met Asn Gln Cys Leu Val Ala Thr Gly Thr His Glu Pro Lys Asn Gln
                   245                 250                 255
   Ser Tyr Met Val Arg Gly Cys Ala Thr Ala Ser Met Cys Gln His Ala
               260                 265                 270
   His Leu Gly Asp Ala Phe Ser Met Asn His Ile Asp Val Ser Cys Cys
           275                 280                 285
   Thr Lys Ser Gly Cys Asn His Pro Asp Leu Asp Val Gln Tyr Arg Ser
       290                 295                 300
   Gly Ala Ala Pro Gln Pro Gly Pro Ala His Leu Ser Leu Thr Ile Thr
   305                 310                 315                 320
   Leu Leu Met Thr Ala Arg Leu Trp Gly Gly Thr Leu Leu Trp Thr
                   325                 330                 335